# EUROPEAN PATENT APPLICATION

(11) **EP 4 644 386 A1**
(43) Date of publication of application: **05.11.2025**
(21) Application number: 24852387.0
(22) Date of filing: 09.08.2024
(51) Int. Cl.: C07D 403/04, A61K 31/4178, A61K 31/4188, A61P 35/00, C07D 487/04, C07D 403/14, C07D 401/04, C07D 401/14

(54) **NOVEL COMPOUND, AND PHARMACEUTICAL COMPOSITION FOR PREVENTION OR TREATMENT OF CANCER OR TUMORS COMPRISING SAME**

(30) Priority: 09.08.2023 KR 20230104339
(71) Applicant: Daewoong Pharmaceutical Co., Ltd., Hwaseong-si, Gyenoggi-do 18623 (KR)
(72) Inventor: KANG, Youngku, Yongin-si, Gyeonggi-do 17028 (KR); AN, Won, Yongin-si, Gyeonggi-do 17028 (KR); LEE, Seul, Yongin-si, Gyeonggi-do 17028 (KR); KWON, Ahreum, Yongin-si, Gyeonggi-do 17028 (KR); KIM, Ji Duck, Yongin-si, Gyeonggi-do 17028 (KR); PARK, Joon Seok, Yongin-si, Gyeonggi-do 17028 (KR)
(74) Representative: Germain Maureau
(86) International application number: PCT/KR2024/011844
(87) International publication number: WO 2025/034034

(57) **Abstract**

The present disclosure relates to a compound represented by the following Chemical Formula 1, or a pharmaceutically acceptable salt thereof, and the compound according to the present disclosure can be used favorably for preventing or treating cancer or tumors. 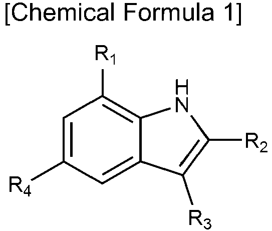 in Chemical Formula 1,
R₁ to R₄ are the same as defined in the detailed description.

## Description

### [TECHNICAL FIELD]

The present disclosure relates to a compound having a novel structure that can be used favorably for preventing or treating cancer or tumors.

### [BACKGROUND ART]

Hippo signaling pathway affects the size and number of cells constituting an organ, and affects organ development and cell growth. In addition, inappropriate regulation of Hippo signaling pathway has been associated with the development of several cancers (breast cancer, head and neck cancer, colon cancer, ovarian cancer, liver cancer, brain cancer, prostate cancer, mesothelioma, sarcoma, etc.).

The Hippo signaling system is composed of several components such as NF2(neurofibromatosis type 2), Mst1/2(mammalian Ste20-like kinases 1 and 2), Lats1/2(large tumor suppressor 1/2), YAP/TAZ(yes-associated protein/WW domain-containing transcription regulator protein), and TEADs(transcriptional enhanced associate domains). Among these, when mutations occur in upstream signaling proteins such as NF2, MST1/2, and LATS1/2, which are tumor suppressors, they induce a persistent binding of YAP/TAZ and TEAD. This promotes transcription by YAP/TAZ and TEAD to induce gene expression related to cancer cell proliferation and tumor formation.

When phosphorylation of Lats1/2 by Mst1/2 is mediated through activation of NF2 which is a tumor suppressor gene, phosphorylation of YAP and TAZ existing in the cytoplasm are promoted. The phosphorylated YAP and TAZ undergoes a ubiquitination process and are then decomposed by proteasome. Therefore, when the Hippo signaling system is turned on, YAP and TAZ are turned off. In contrast, under conditions in which tumor suppressor genes are turned off, i.e. when Hippo signaling is turned off, YAP and TAZ are turned on and translocate to the nucleus, which binds to four proteins of the TEAD family (TEAD1/2/3/4) and induces the expression of target genes such as CTGF (connective tissue growth factor), CYR61(cysteine-rich angiogenic inducer 61), Gli2 (GLI family zinc finger 2), Birc2/5(Baculoviral IAP repeat containing 2/5), FGF(fibroblast growth factor). In this manner, genes turned on by the YAP/TAZ-transcription factor complex regulate cell growth, cell migration, and apoptosis.

Abnormalities in the Hippo signaling system have been found in several carcinomas, and several research results have been reported to develop anticancer drugs targeting YAP-TEAD. Furthermore, the relevance of the Hippo signaling system has been revealed in the process of acquiring resistance after application of existing approved anticancer drugs, and several studies are underway to demonstrate the potential of YAP-TEAD anticancer drugs as combination therapeutics to treat drug resistance. Therefore, there is a need to develop a small molecule inhibitor for treating cancer caused by dysregulation of the Hippo signaling system.

In this regard, the present inventors have studied medicines that can be used favorably for treating cancer in which the regulation of Hippo signaling is turned off, and as a result, found that the compound according to the present disclosure described hereinafter binds to the palmitate binding site that mediates TEAD palmitoylation, and also inhibits the in vitro growth of a Hippo pathway mutated cell line. Through this, it has been found that the YAP-TEAD inhibitor according to the present disclosure can be used favorably for treating cancer or tumors in which Hippo signaling is turned off, and completed the present disclosure.

### [DETAILED DESCRIPTION OF THE INVENTION]

### [Technical Problem]

It is an object of the present disclosure to provide a compound having a novel structure that can be used favorably for preventing or treating cancer or tumors.

It is another object of the present disclosure to provide a pharmaceutical composition for the prevention or treatment of cancer or tumors comprising the above compound.

### [Technical Solution]

In order to achieve the above object, provided herein is a compound represented by the following Chemical Formula 1, or a pharmaceutically acceptable salt thereof: 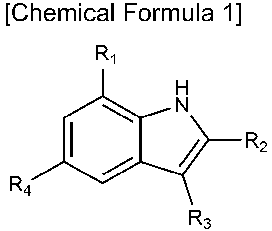 in Chemical Formula 1,
R₁ is a C₆₋₁₀ aryl; or a 5- or 6-membered heterocycle containing 1 to 4 heteroatoms each independently selected from the group consisting of N, O and S,
   wherein the R₁ is unsubstituted; or substituted with halogen, a C₁₋₄ alkyl, a C₁₋₄ thioalkyl, a C₁₋₄ haloalkyl, a C₁₋₄ alkoxy, a C₁₋₄ thioalkoxy, a C₁₋₄ haloalkoxy, a C₂₋₄ alkenyl, a C₂₋₄ alkynyl, a C₃₋₆ cycloalkyl, amino, nitro, cyano, a (C₁₋₄ alkyl)amino, or a di(C₁₋₄ alkyl)amino; or is fused with a C₃₋₆ cycloalkyl ring,
R₂ is a C₆₋₁₀ aryl; a C₃₋₆ cycloalkenyl; or a 5- or 6-membered heterocycle containing 1 to 4 heteroatoms each independently selected from the group consisting of N, O and S,
   wherein the R₂ is unsubstituted; or substituted with 1 to 3 substituents each independently selected from the group consisting of halogen, a C₁₋₄ alkyl, a C₁₋₄ thioalkyl, a C₁₋₄ haloalkyl, a C₁₋₄ alkoxy, a C₁₋₄ thioalkoxy, a C₁₋₄ haloalkoxy, a C₂₋₄ alkenyl, a C₂₋₄ alkynyl, a C₃₋₆ cycloalkyl, amino, nitro, cyano, a (C₁₋₄ alkyl)amino, and a di(C₁₋₄ alkyl)amino,
R₃ is hydrogen or halogen,
R₄ is -NHCO-R', -NHSO₂-R', -COO-R', -CONH-R', or 1H-pyrrole-2,5-dione-1-yl,
   wherein the R' is hydrogen, a C₁₋₄ alkyl, a C₂₋₄ alkynyl, or a substituent represented by the following Chemical Formula 2: 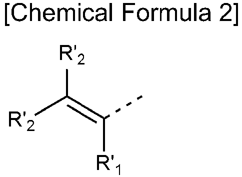
in Chemical Formula 2,
R'₁ is hydrogen, halogen, or cyano, and
R'₂ is each independently hydrogen, a C₆₋₁₀ aryl, -CH₂-NH₂, -CH₂-NH(C₁₋₄ alkyl), or -CH₂-N(C₁₋₄ alkyl)₂.

Preferably, R₁ is phenyl, pyridinyl, pyrimidinyl, imidazolyl, pyrazolyl, triazolyl, tetrazolyl, oxadiazolyl, pyrrolidinyl, imidazolyl, pyrazolyl, oxazolyl, isoxazolyl, thiazolyl, or isothiazolyl,
wherein the R₁ is unsubstituted; or substituted with halogen, a C₁₋₄ alkyl, a C₁₋₄ thioalkyl, a C₁₋₄ haloalkyl, a C₁₋₄ alkoxy, a C₁₋₄ haloalkoxy, amino, nitro, cyano, a (C₁₋₄ alkyl)amino, or a di(C₁₋₄ alkyl)amino; or is fused with a C₃₋₆ cycloalkyl ring.

Preferably, R₁ is imidazolyl, imidazolyl fused with cyclopentane, pyrazolyl, or pyridinyl,
wherein the R₁ is unsubstituted; or substituted with methyl, ethyl, fluoromethyl, difluoromethyl, or trifluoromethyl.

Preferably, R₂ is phenyl, pyridinyl, pyrimidinyl, imidazolyl, pyrazolyl, triazolyl, tetrazolyl, oxadiazolyl, pyrrolidinyl, imidazolyl, pyrazolyl, oxazolyl, isoxazolyl, thiazolyl, or isothiazolyl,
wherein the R₂ is unsubstituted; or substituted with halogen, a C₁₋₄ alkyl, a C₁₋₄ thioalkyl, a C₁₋₄ haloalkyl, a C₁₋₄ alkoxy, a C₁₋₄ haloalkoxy, amino, nitro, cyano, a (C₁₋₄ alkyl)amino, or a di(C₁₋₄ alkyl)amino.

Preferably, R₂ is phenyl, pyridinyl, or cyclohexenyl,
wherein the R₂ is unsubstituted; or substituted with 1 to 3 substituents each independently selected from the group consisting of fluoro, chloro, brormo, methyl, trifluoromethyl, and trifluoromethoxy.

Preferably, R₃ is hydrogen, or chloro.

Preferably, R₄ is -NHCO-R', -NHSO₂-R', -COO-R', -CONH-R', or 1H-pyrrole-2,5-dione-1-yl;
wherein the R' is hydrogen; a C₁₋₄ alkyl; a C₂₋₄ alkenyl which is unsubstituted, or substituted with 1 or 2 substituents each independently selected from the group consisting of phenyl, fluoro, and cyano; or a C₂₋₄ alkynyl.

Preferably, R₄ is -NHCO-CH=CH₂, -NHCO-CF=CH₂, -NHCO-C(CN)=CH(phenyl), -NHCO-C≡C-CH₃, -NHSO₂-CH₃, -NHSO₂-CH=CH₂, -COOH, - CONH-CH₃, or 1H-pyrrole-2,5-dione-1-yl,
Preferably, the Chemical Formula 1 is represented by the following Chemical Formula 3: 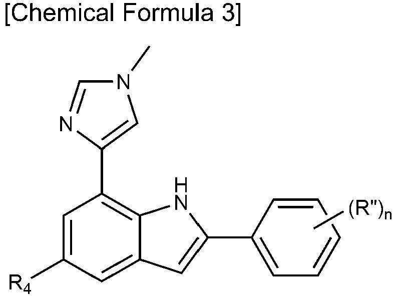 in Chemical Formula 3,
R" is each independently hydrogen, halogen, a C₁₋₄ alkyl, or a C₁₋₄ haloalkyl,
n is an integer of 1 to 3, and
R₄ is -NHCO-CH=CH₂, or -COOH.

Representative examples of the compounds represented by Chemical Formula 1 are as follows:
1) N-(2-(4-chlorophenyl)-7-(1-methyl-1H-imidazol-4-yl)-1H-indol-5-yl)acrylamide,
2) N-(7-(1-methyl-1H-imidazol-4-yl)-2-(4-(trifluoromethyl)phenyl)-1H-indol-5-yl)acrylamide,
3) N-(2-(4-fluorophenyl)-7-(1-methyl-1H-imidazol-4-yl)-1H-indol-5-yl)acrylamide,
4) N-(7-(1-methyl-1H-imidazol-4-yl)-2-phenyl-1H-indol-5-yl)acrylamide,
5) N-(2-(2,4-difluorophenyl)-7-(1-methyl-1H-imidazol-4-yl)-1H-indol-5-yl)acrylamide,
6) N-(7-(1-methyl-1H-imidazol-4-yl)-2-(3,4,5-trifluorophenyl)-1H-indol-5-yl)acrylamide,
7) N-(7-(1-(difluoromethyl)-1H-imidazol-4-yl)-2-(4-fluorophenyl)-1H-indol-5-yl)acrylamide,
8) N-(2-(3,4-dichlorophenyl)-7-(1-methyl-1H-imidazol-4-yl)-1H-indol-5-yl)acrylamide,
9) N-(2-(3,4-difluorophenyl)-7-(1-methyl-1H-imidazol-4-yl)-1H-indol-5-yl)acrylamide,
10) N-(2-(3-chlorophenyl)-7-(1-methyl-1H-imidazol-4-yl)-1H-indol-5-yl)acrylamide,
11) N-(2-(4-chlorophenyl)-7-(1-(difluoromethyl)-1H-imidazol-4-yl)-1H-indol-5-yl)acrylamide,
12) N-(2-(4-chloro-2-fluorophenyl)-7-(1-methyl-1H-imidazol-4-yl)-1H-indol-5-yl)acrylamide,
13) N-(2-cyclohexenyl-7-(1-methyl-1H-imidazol-4-yl)-1H-indol-5-yl)acrylamide,
14) N-(2-(4-fluoro-3-methylphenyl)-7-(1-methyl-1H-imidazol-4-yl)-1H-indol-5-yl)acrylamide,
15) N-(7-(1-methyl-1H-imidazol-4-yl)-2-p-tolyl-1H-indol-5-yl)acrylamide,
16) N-(7-(1-methyl-1H-imidazol-4-yl)-2-(3-(trifluoromethyl)phenyl)-1H-indol-5-yl)acrylamide,
19) N-(7-(1-methyl-1H-imidazol-4-yl)-2-(3-(trifluoromethyl)phenyl)-1H-indol-5-yl)methanesulfonamide,
20) N-(2-(4-chloro-3-fluorophenyl)-7-(1-methyl-1H-imidazol-4-yl)-1H-indol-5-yl)acrylamide,
21) N-(2-(4-chlorophenyl)-7-(1-methyl-1H-imidazol-4-yl)-1H-indol-5-yl)methanesulfonamide,
22) N-(2-(4-chlorophenyl)-7-(1-methyl-1H-imidazol-4-yl)-1H-indol-5-yl)-2-fluoroacrylamide,
23) N-(3-chloro-2-(4-chlorophenyl)-7-(1-methyl-1H-imidazol-4-yl)-1H-indol-5-yl)acrylamide,
24) (E)-N-(2-(4-chlorophenyl)-7-(1-methyl-1H-imidazol-4-yl)-1H-indol-5-yl)-2-cyano-3-phenylacrylamide,
25) N-(2-(4-chlorophenyl)-7-(1-methyl-1H-imidazol-4-yl)-1H-indol-5-yl)but-2-ynamide,
26) N-(2-(3,4-dichlorophenyl)-7-(1-methyl-1H-imidazol-4-yl)-1H-indol-5-yl)ethenesulfonamide,
27) 1-(2-(3,4-dichlorophenyl)-7-(1-methyl-1H-imidazol-4-yl)-1H-indol-5-yl)-1H-pyrrole-2,5-dione,
28) N-(2-(4-chlorophenyl)-7-(1-methyl-1H-pyrazol-3-yl)-1H-indol-5-yl)acrylamide,
29) N-(2-(3,4-dichlorophenyl)-7-(1-methyl-1H-pyrazol-3-yl)-1H-indol-5-yl)acrylamide,
30) N-(2-(3,4-dichlorophenyl)-7-(pyridin-2-yl)-1H-indol-5-yl)acrylamide,
31) N-(2-(4-fluorophenyl)-7-(pyridin-2-yl)-1H-indol-5-yl)acrylamide,
32) N-(2-(4-fluorophenyl)-7-(1-methyl-1H-pyrazol-3-yl)-1H-indol-5-yl)acrylamide,
33) N-(2-(2,4-difluorophenyl)-7-(1-methyl-1H-pyrazol-3-yl)-1H-indol-5-yl)acrylamide,
34) N-(2-(5-chloropyridin-2-yl)-7-(1-methyl-1H-imidazol-4-yl)-1H-indol-5-yl)acrylamide,
35) 2-(4-fluorophenyl)-7-(1-methyl-1H-imidazol-4-yl)-1H-indole-5-carboxylic acid,
36) 7-(1-methyl-1H-imidazol-4-yl)-2-(4-(trifluoromethyl)phenyl)-1H-indole-5-carboxylic acid,
37) 2-(4-chlorophenyl)-7-(1-methyl-1H-imidazol-4-yl)-1H-indole-5-carboxylic acid,
38) 2-(3,4-dichlorophenyl)-7-(1-methyl-1H-imidazol-4-yl)-1H-indole-5-carboxylic acid,
39) 2-(4-chloro-3-fluorophenyl)-7-(1-methyl-1H-imidazol-4-yl)-1H-indole-5-carboxylic acid,
40) 2-(4-chlorophenyl)-N-methyl-7-(1-methyl-1H-imidazol-4-yl)-1H-indole-5-carboxamide,
41) N-(7-(1-methyl-1H-imidazol-4-yl)-2-(3,4,5-trichlorophenyl)-1H-indol-5-yl)acrylamide,
42) N-(2-(3-fluorophenyl)-7-(1-methyl-1H-imidazol-4-yl)-1H-indol-5-yl)acrylamide,
43) 2-(3,4-difluorophenyl)-7-(1-methyl-1H-imidazol-4-yl)-1H-indole-5-carboxylic acid,
44) N-(2-(3-chloro-4-fluorophenyl)-7-(1-methyl-1H-imidazol-4-yl)-1H-indol-5-yl)acrylamide,
45) N-(2-(2,4-dichlorophenyl)-7-(1-methyl-1H-imidazol-4-yl)-1H-indol-5-yl)acrylamide,
46) N-(2-(4-chloro-3,5-difluorophenyl)-7-(1-methyl-1H-imidazol-4-yl)-1H-indol-5-yl)acrylamide,
47) N-(2-(4-chloro-2,6-difluorophenyl)-7-(1-methyl-1H-imidazol-4-yl)-1H-indol-5-yl)acrylamide,
48) N-(7-(1-methyl-1H-imidazol-4-yl)-2-(4-(trifluoromethoxy)phenyl)-1H-indol-5-yl)acrylamide,
49) N-(2-(4-chlorophenyl)-7-(4-methylthiazol-2-yl)-1H-indol-5-yl)acrylamide,
50) N-(7-(1-ethyl-1H-imidazol-4-yl)-2-(3,4,5-trifluorophenyl)-1H-indol-5-yl)acrylamide,
51) N-(7-(1-ethyl-1H-imidazol-4-yl)-2-(4-fluorophenyl)-1H-indol-5-yl)acrylamide, and
52) N-(2-(4-bromophenyl)-7-(1-methyl-1H-imidazol-4-yl)-1H-indol-5-yl)acrylamide.

In addition, the compounds of the present disclosure may exist in the form of salts, especially pharmaceutically acceptable salts. As salts, salts commonly used in the art, such as acid addition salts formed by pharmaceutically acceptable free acids can be used without limitation. The term "pharmaceutically acceptable salt" as used herein refers to any organic or inorganic addition salt of the compound represented by Chemical Formula 1, whose concentration is relatively non-toxic and harmless to ae patient and activates effectively and whose side effects do not degrade the beneficial efficacy of the above compound.

As the free acid, an organic acid and an inorganic acid can be used. Examples of the inorganic acids include hydrochloric acid, phosphoric acid, sulfuric acid, nitric acid, tartaric acid and the like. Examples of the organic acids include methanesulfonic acid, p-toluenesulfonic acid, acetic acid, trifluoroacetic acid, maleic acid, succinic acid, oxalic acid, benzoic acid, tartaric acid, fumaric acid, mandelic acid, propionic acid, citric acid, lactic acid, glycollic acid, gluconic acid, galacturonic acid, glutamic acid, glutaric acid, glucuronic acid, aspartic acid, ascorbic acid, carbonic acid, vanillic acid, hydroiodic acid and the like, but are not limited thereto.

In addition, a pharmaceutically acceptable metal salt can be obtained by a conventional method using a base. For example, a compound represented by Chemical Formula 1 is dissolved in an excessive amount of an alkali metal hydroxide or an alkaline earth metal hydroxide solution, the non-soluble salt is filtered, and then the filtrate is evaporated and dried to obtain a pharmaceutically acceptable metal salt. At this time, it is particularly preferable to prepare a sodium salt, a potassium salt or a calcium salt as the metal salt.

Further, a pharmaceutically unacceptable salt or solvate of the compound represented by Chemical Formula 1 may be used as an intermediate when preparing the compound represented by Chemical Formula 1, or the pharmaceutically acceptable salt or the solvate thereof.

Meanwhile, the compound represented by Chemical Formula 1 can be prepared as shown in the following Reaction Scheme 1 or 2.

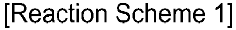

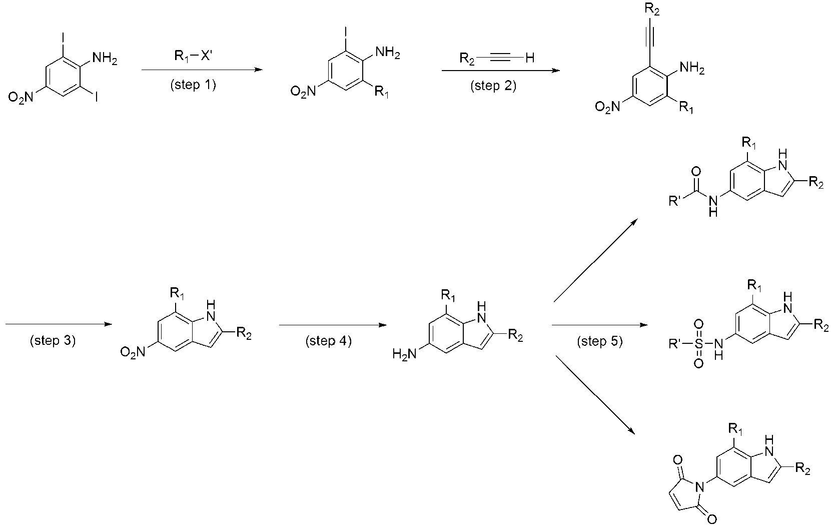

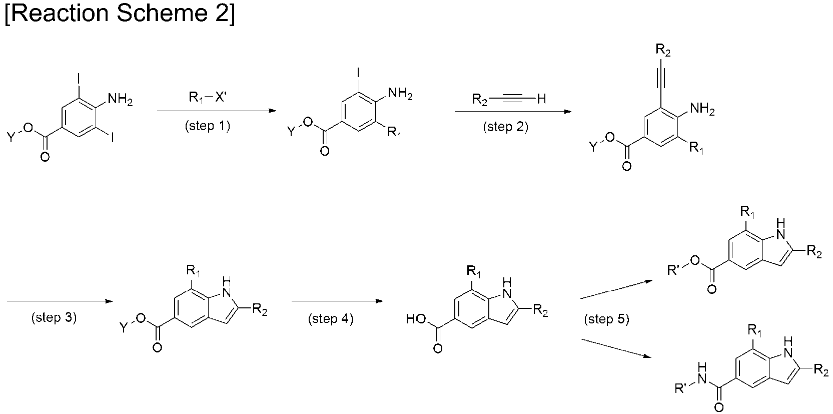

In Reaction Schemes 1 and 2, the remaining substituents except for Y are the same as defined above, and Y is a C₁₋₄ alkyl, more preferably methyl or ethyl.

Each step 1 of Reaction Schemes 1 and 2 is a Stille coupling reaction, and step 2 is a Sonogashira coupling reaction, wherein the order of step 1 and step 2 may be changed depending on the reactivity of each reactant (see Example 28). At this time, X' is a substituent for the Stille coupling reaction, and may be -SnBu₃, or -B(OH)₂ for Suzuki reaction, but is not limited thereto.

Each step 3 of Reaction Schemes 1 and 2 is a reaction for synthesizing indole through a cyclization reaction, which reaction can be carried out under basic conditions, but is not limited thereto.

Step 4 of Reaction Scheme 1 is a reaction for reducing a nitro group, which reaction can be carried out using iron, but is not limited thereto.

Step 5 of Reaction Scheme 1 is a reaction between an amine group and a carbonyl group or a sulfonyl group according to R₄.

Step 4 of Reaction Scheme 2 is a hydrolysis reaction, which can be carried out under basic conditions, but is not limited thereto.

Step 5 of Reaction Scheme 2 may be a coupling reaction between a nucleophile and a carbonyl group according to R₄, but is not limited thereto.

Meanwhile, Reaction Schemes 1 and 2 are the cases where R₃ in Chemical Formula 1 of the present disclosure is hydrogen, and if R₃ is not hydrogen, after step 3 but before step 4, indole derivative is synthesized by cyclization reactions and then an R₃ substitution reaction is carried out to substitute R₃.

The preparation method may be more specifically described in the Example Examples described hereinafter.

Further provided is a pharmaceutical composition comprising the compound represented by Chemical Formula 1, or a pharmaceutically acceptable salt thereof. Specifically, provided is a pharmaceutical composition for the prevention or treatment of cancer or tumors, comprising the compound represented by Chemical Formula 1, or a pharmaceutically acceptable salt thereof as an active ingredient.

The terms "cancer" or "tumor" refer to the presence, e.g., in a subject, of cells possessing characteristics typical of cancer-causing cells, such as uncontrolled proliferation, immortality, metastatic potential, rapid growth and proliferation rate, decreased cell death/apoptosis. Cancer cells often include solid tumors, such as a sarcomas or a carcinomas. The sarcomas includes alveolar rhabdomyosarcoma, alveolar soft part sarcoma, ameloblastoma, angiosarcoma, chondrosarcoma, chordoma, clear cell sarcoma, dedifferentiated liposarcoma, desmoid, connective tissue small round cell tumor, embryonal rhabdomyosarcoma, epithelioid fibrosarcoma, epithelioid hemangioendothelioma, epithelioid sarcoma, esthesioneuroblastoma, Ewing sarcoma, extrarenal rhabdoid tumor, extraskeletal myxoid chondrosarcoma, extrasketetal osteosarcoma, fibrosarcoma, giant cell tumor, hemangiopericytoma, infantile fibrosarcoma, inflammatory myofibroblastic tumor, Kaposi sarcoma, osteosarcoma, liposarcoma, liposarcoma of bone, malignant fibrous histiocytoma, malignant fibrous histiocytoma of bone, malignant mesenchymoma, malignant peripheral nerve sheath tumor, mesenchymal chondrosarcoma, myxofibrosarcoma; myxoid liposarcoma, myxoinflammatory fibroblastic sarcoma, osteosarcoma, parosteal osteosarcoma, periosteal osteosarcoma, pleomorphic liposarcoma, pleomorphic rhabdomyosarcoma, extraskeletal Ewing tumor, rhabdomyosarcoma, round cell liposarcoma, small cell osteosarcoma, solitary fibrous tumor, synovial sarcoma, and telangiectatic osteosarcoma. The carcinoma includes an adenocarcinoma, squamous cell carcinoma, adenosquamous carcinoma, anaplastic carcinoma, large cell carcinoma, small cell carcinoma, anal cancer, appendix cancer; bile duct cancer, bladder cancer, brain tumor, breast cancer, cervical cancer, colon cancer, cancer of Unknown Primary, esophageal cancer, eye cancer, primary fallopian tube cancer, digestive organ cancer, kidney cancer, liver cancer, lung cancer, medulloblastoma, malignant melanoma, oral cancer, ovarian cancer, pancreatic cancer, parathyroid cancer, penile tumor, pituitary tumor, prostate cancer, rectal cancer, skin cancer, stomach cancer, testicular cancer, throat cancer, thyroid cancer, uterine cancer, vaginal cancer, and vulvar cancer. In some embodiments, the carcinoma is breast cancer. In some embodiments, the cancer used in this application include may include uveal melanoma, mesothelioma, esophageal cancer, liver cancer, breast cancer, hepatocellular carcinoma, lung adenocarcinomas, glioma, colorectal cancer, colon cancer, stomach cancer, medulloblastoma, ovarian cancer, esophageal squamous cell carcinoma, sarcoma, Ewing sarcoma, head and neck cancer, prostate cancer, and meningioma.

Cancer also includes non-solid tumors, such as blood cancers, wherein the cancer cells are derived from bone marrow. The blood cancer includes leukemia, lymphoma, myeloma, non-Hodgkin lymphoma, Hodgkin lymphoma, T-cell malignancy, or B-cell malignancy. In particular, the T-cell malignancy includes mature T-cell lymphoma not elsewhere classified, anaplastic large cell lymphoma, angioimmunoblastic lymphoma, cutaneous T-cell lymphoma, adult T-cell leukemia, blastic natural killer cell lymphoma, platelet gamma-delta T-cell lymphoma, and lymphoblastic lymphoma. The B-cell malignancy includes chronic lymphocytic leukemia, small lymphocytic lymphoma, follicular lymphoma, diffuse large B-cell lymphoma, mantle cell lymphoma, Waldenström macroglobulinemia, head and neck tumor, nodal marginal zone B cell lymphoma, Burkitt's lymphoma, primary mediastinal B-cell lymphoma, immune large cell lymphoma, progenitor B-lymphoblastic lymphoma, prolymphocytic leukemia, lymphoplasmacytic lymphoma, nasal peripheral zone lymphoma, plasma cell myeloma, plasmacytoma, intravascular large B-cell lymphoma, primary effusion lymphoma, or lymphomatoid granulomatosis, but is not limited thereto.

In some embodiments, it includes recurrent or refractory cancer, wherein the cancer is a solid tumor. The recurrent or incurable cancer includes adenocarcinoma, squamous cell carcinoma, adenosquamous cell carcinoma, anaplastic carcinoma, large cell carcinoma, small cell carcinoma, biliary tract cancer, bladder cancer, brain tumor, breast cancer, cervical cancer, colorectal cancer, esophageal cancer, ophthalmic tumor, primary fallopian tube cancer, kidney cancer, liver cancer, lung cancer, medulloblastoma, malignant melanoma, oral cancer, ovarian cancer, pancreatic cancer, penile cancer, pituitary tumor, prostate cancer, rectal cancer, skin cancer, stomach cancer, testis cancer, throat cancer, thyroid cancer, uterine cancer, vaginal cancer, and vulvar cancer.

As used herein, the term "prevention" refers to any act to delay or inhibit occurrence, spread or recurrence of the above-mentioned diseases by administration of the composition of the present disclosure, and "treatment" refers to any act to improve or change the symptoms of the above diseases for the better by administration of the composition of the present disclosure.

The pharmaceutical composition according to the present disclosure can be formulated in types for oral or parenteral administrations according to a standard pharmaceutical practice. These formulations may contain additives such as pharmaceutically acceptable carrier, adjuvant or diluent in addition to the active ingredient.

Suitable carriers include, for example, physiological saline, polyethylene glycol, ethanol, vegetable oil, and isopropyl myristate and the like. Diluents include, for example, lactose, dextrose, sucrose, mannitol, sorbitol, cellulose and/or glycine and the like, but are not limited thereto. Further, the compounds of the present disclosure can be dissolved in oils, propylene glycol or other solvents commonly used in the preparation of injection solutions. Furthermore, the compounds of the present disclosure can be formulated in ointments or creams for topical application.

A preferred dose of the compound of the present disclosure may be varied according to the condition and weight of a patient, the severity of a disease, the type of a drug, and the route and duration of administration, but it may be suitably selected by those skilled in the art. In order to achieve the desirable effects, however, the compound of the present disclosure may be administrated daily at a dose of 0.0001 to 100 mg/kg (body weight), and preferably 0.001 to 100 mg/kg (body weight). The administration may be performed once a day or in divided doses each day through an oral or parenteral route.

Depending on the method of administration, the pharmaceutical composition may contain the compound of the present disclosure in an amount of 0.001 to 99% by weight, preferably 0.01 to 60% by weight.

The pharmaceutical composition according to the present disclosure may be administered to mammals such as a rat, a mouse, a domestic animal, a human, through various routes. The administration may be carried out through all possible methods, for example, oral, rectal, intravenous, intramuscular, subcutaneous, intra-endometrial, intracerebroventricular injection.

### [Advantageous Effects]

A compound represented by Chemical Formula 1 of the present disclosure, or a pharmaceutically acceptable salt thereof can be used favorably for preventing or treating cancer or tumors.

### [BRIEF DESCRIPTION OF THE DRAWINGS]

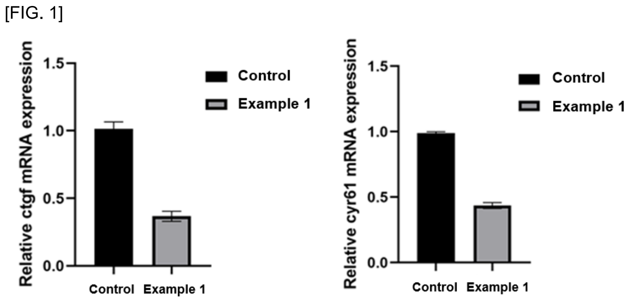

FIG. 1 shows the results of Experimental Example 3, and shows the expression regulatory properties of two TEAD target genes in Example 1 using real-time PCR.

### [DETAILED DESCRIPTION OF THE EMBODIMENTS]

Hereinafter, preferred examples are presented to assist in the understanding of the present disclosure. However, the following examples are for illustrative purposes only, and should not be construed as limiting the scope of the present disclosure to these examples.

### Example 1: Preparation of N-(2-(4-chlorophenyl)-7-(1-methyl-1H-imidazol-4-yl)-1H-indol-5-yl)acrylamide

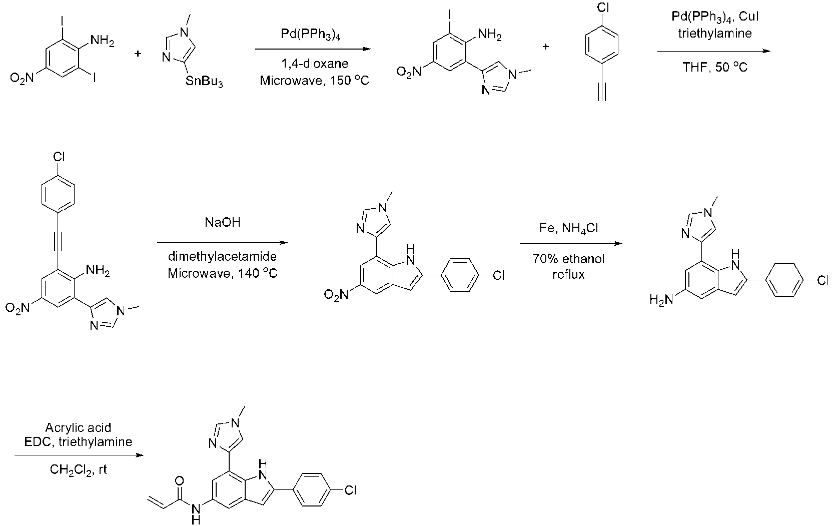

### (Step 1)

In a sealed tube, 2,6-diiodo-4-nitroaniline (2.56 mmol, 1 g, 1.0 eq) was dissolved in 1,4-dioxane (10 mL). 1-Methyl-4-(tributylstannyl)-1H-imidazole (2.56 mmol, 0.95 g, 1.0 eq) and Pd(PPh₃)₄ (0.25 mmol, 0.29 g, 0.1 eq) were sequentially added thereto, and then reacted in a microwave reactor at 150°C for 1 hour. After the reaction was completed, the reaction mixture was extracted with ethyl acetate and ammonium chloride aqueous solution, concentrated, and then solidified with tert-butyl methyl ether to give 2-iodo-6-(1-methyl-1H-imidazol-4-yl)-4-nitroaniline (0.57 g, yield: 64%).

### (Step 2)

2-Iodo-6-(1-methyl-1H-imidazol-4-yl)-4-nitroaniline (0.58 mmol, 0.2 g, 1.0 eq), Pd(PPh₃)₄ (0.058 mmol, 0.067 g, 0.1 eq), copper iodide (0.03 mmol, 0.0057 g, 0.05 eq), triethylamine (1.7 mmol, 0.24 mL, 2.9 eq), and tetrahydrofuran (2 mL) were sequentially added, and then ultrasonic degassing was carried out under a nitrogen atmosphere for 10 minutes. 1-Chloro-4-ethynylbenzene (0.69 mmol, 0.094 g, 1.2 eq) was added thereto and reacted at 50°C for 2 hours. After the reaction was completed, the reaction mixture was extracted with ethyl acetate and ammonium chloride aqueous solution, concentrated, and then solidified with tert-butyl methyl ether to give 2-((4-chlorophenyl)ethynyl)-6-(1-methyl-1H-imidazol-4-yl)-4-nitroaniline (0.14 g, yield: 68%).

### (Step 3)

2-((4-Chlorophenyl)ethynyl)-6-(1-methyl-1H-imidazol-4-yl)-4-nitroaniline (0.39 mmol, 0.14 g, 1.0 eq), sodium hydroxide (1.95 mmol, 0.078 g, 5.0 eq) and dimethylacetamide (10 mL) were sequentially added to a microwave vial, and then reacted at 150°C for 15 minutes. After the reaction was completed, the reaction mixture was extracted with ethyl acetate and ammonium chloride aqueous solution, concentrated, and then solidified with tert-butyl methyl ether to give 2-(4-chlorophenyl)-7-(1-methyl-1H-imidazol-4-yl)-5-nitro-1H-indole (0.079 g, yield: 57%).

### (Step 4)

2-(4-Chlorophenyl)-7-(1-methyl-1H-imidazol-4-yl)-5-nitro-1H-indole (0.22 mmol, 0.079 g, 1.0 eq), iron (1.0 mmol, 0.055 g, 4.54 eq), ammonium chloride (0.22 mmol, 0.012 g, 1.0 eq), and 10 mL of 70% ethanol (ethanol: distilled water = 7:3 (v:v)) were sequentially added, and the reactants were reacted overnight at 60°C. After the reaction was completed, iron and ethanol were removed, extracted with an aqueous ammonium chloride solution, and concentrated, and 2-(4-chlorophenyl)-7-(1-methyl-1H-imidazol-4-yl)-1H-indol-5-amine was used in the next reaction without separation and purification.

### (Step 5)

2-(4-Chlorophenyl)-7-(1-methyl-1H-imidazol-4-yl)-1H-indol-5-amine (0.1 mmol, 0.032 g, 1.0 eq), N-(3-dimethylaminopropyl)-N-ethylcarbodiimide hydrochloride (0.3 mmol, 0.057 g, 3.0 eq), and 1 mL of dichloromethane was sequentially added to a flask. 0.05 mL of triethylamine and acrylic acid (0.12 mmol, 0.0086 mL, 1.2 eq) were added thereto, and then reacted overnight at room temperature. After the reaction was completed, dichloromethane was removed, and purified by a column chromatography to give the title compound (9.4 mg, yield: 25%).

¹H NMR (400 MHz, CDCl₃) δ 11.14 s, 1H), 7.77 (s, 1H), 7.70-7.68 (m, 2H), 7.55-7.54 (m, 2H), 7.44-7.40 (m, 3H), 7.24-7.25 (m, 1H), 6.76 (d, J = 2.2 Hz, 1H), 6.46 (d, J = 16.0, 1H), 6.33-6.26 (m, 1H), 5.78 (d, J = 12.0, 1H), 3.79 (s, 3H)

### Example 2: Preparation of N-(7-(1-methyl-1H-imidazol-4-yl)-2-(4-(trifluoromethyl)phenyl)-1H-indol-5-yl)acrylamide

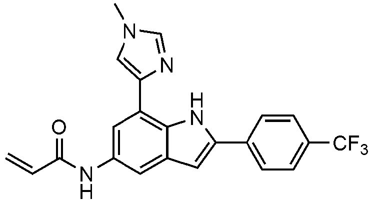

The title compound (8.6 mg, yield: 21%) was obtained in the same manner as in Example 1, except that 1-trifluoromethyl-4-ethynylbenzene was used instead of 1-chloro-4-ethynylbenzene in step 2 of Example 1.

¹H NMR (400 MHz, CDCl₃) δ 11.28 (s, 1H), 7.87-7.85 (m, 2H), 7.80 (s, 1H), 7.70-7.68 (m, 2H), 7.60-7.58 (m, 2H), 7.41 (s, 1H), 7.25 (s, 1H), 6.88 (s, 1H), 6.46 (d, J = 12.0, 1H), 6.32-6.27 (m, 1H), 5.78 (d, J = 8.0, 1H), 3.80 (s, 3H).

### Example 3: Preparation of N-(2-(4-fluorophenyl)-7-(1-methyl-1H-imidazol-4-yl)-1H-indol-5-yl)acrylamide

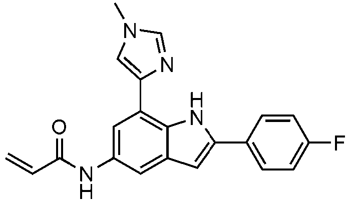

The title compound (8.6 mg, yield: 21%) was obtained in the same manner as in Example 1, except that 1-ethynyl-4-fluorobenzene was used instead of 1-chloro-4-ethynylbenzene in step 2 of Example 1.

¹H NMR (500 MHz, DMSO) δ 11.13 (s, 1H), 10.08 (s, 1H), 7.89-7.86 (m, 4H), 7.76 (s, 1H), 7.68 (d, J = 1.6 Hz, 1H), 7.35 (t, J = 8.8 Hz, 2H), 6.92 (d, J = 2.2 Hz, 1H), 6.49 (dd, J = 16.9, 10.1 Hz, 1H), 6.26 (dd, J = 17.0, 1.8 Hz, 1H), 5.74 (dd, J = 10.1, 1.8 Hz, 1H), 3.80 (s, 3H).

### Example 4: Preparation of N-(7-(1-methyl-1H-imidazol-4-yl)-2-phenyl-1H-indol-5-yl)acrylamide

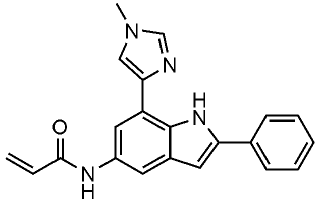

The title compound (11.9 mg, yield: 35%) was obtained in the same manner as in Example 1, except that ethynylbenzene was used instead of 1-chloro-4-ethynylbenzene in step 2 of Example 1.

¹H NMR (500 MHz, DMSO) δ 11.13 (s, 1H), 10.08 (s, 1H), 7.89-7.86 (m, 4H), 7.76 (s, 1H), 7.68 (d, J = 1.6 Hz, 1H), 7.35 (t, J = 8.8 Hz, 2H), 6.92 (d, J = 2.2 Hz, 1H), 6.49 (dd, J = 16.9, 10.1 Hz, 1H), 6.26 (dd, J = 17.0, 1.8 Hz, 1H), 5.74 (dd, J = 10.1, 1.8 Hz, 1H), 3.80 (s, 3H).

### Example 5: Preparation of N-(2-(2,4-difluorophenyl)-7-(1-methyl-1H-imidazol-4-yl)-1H-indol-5-yl)acrylamide

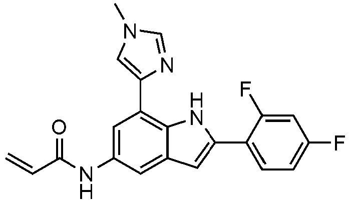

The title compound (6.0 mg, yield: 16%) was obtained in the same manner as in Example 1, except that 1-ethynyl-2,4-difluorobenzene was used instead of 1-chloro-4-ethynylbenzene in step 2 of Example 1.

¹H NMR (500 MHz, DMSO) δ 11.48 (s, 1H), 10.08 (s, 1H), 8.04-7.99 (m, 1H), 7.88 (d, J = 10.0 Hz, 2H), 7.73-7.69 (m, 2H), 7.49-7.44 (m, 1H), 7.27 (td, J = 8.5, 2.0 Hz, 1H), 6.98 (d, J = 1.5 Hz, 1H), 6.50 (dd, J = 17.0, 10.0 Hz, 1H), 6.27 (dd, J = 17.0, 2.0 Hz, 1H), 5.74 (dd, J = 10.0, 2.0 Hz, 1H), 3.79 (s, 3H).

### Example 6: Preparation of N-(7-(1-methyl-1H-imidazol-4-yl)-2-(3,4,5-trifluorophenyl)-1H-indol-5-yl)acrylamide

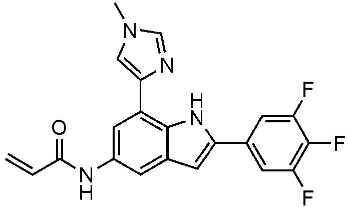

The title compound (17.8 mg, yield: 45%) was obtained in the same manner as in Example 1, except that 5-ethynyl-1,2,3-trifluorobenzene was used instead of 1-chloro-4-ethynylbenzene in step 2 of Example 1.

¹H NMR (500 MHz, CDCl₃) δ 11.21 (s, 1H), 7.82 (s, 1H), 7.61-7.60 (m, 2H), 7.42-7.29 (m, 3H), 7.31 (s, 1H), 6.76 (s, 1H), 6.32 (dd, J = 16.6, 10.4 Hz, 1H), 5.80 (d, J = 10.1 Hz, 1H), 3.82 (s, 3H).

### Example 7: Preparation of N-(7-(1-(difluoromethyl)-1H-imidazol-4-yl)-2-(4-fluorophenyl)-1H-indol-5-yl)acrylamide

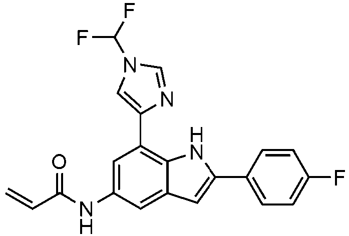

The title compound (9.1 mg, yield: 23%) was obtained in the same manner as in Example 1, except that 1-(difluoromethyl)-4-(tributylstannyl)-1H-imidazole was used instead of 1-methyl-4-(tributylstannyl)-1H-imidazole in step 1 of Example 1, and 1-ethynyl-4-fluorobenzene was used instead of 1-chloro-4-ethynylbenzene in Step 2 of Example 1.

¹H NMR (500 MHz, DMSO) δ 10.85 (s, 1H), 10.05 (s, 1H), 8.42 (s, 1H), 8.26 (s, 1H), 7.97-7.88 (m, 3H), 7.94 (CHF₂, t, J = 60 Hz, 1H), 7.83 (s, 1H), 7.34 (t, J = 8.7 Hz, 2H), 6.93 (s, 1H), 6.50 (dd, J = 16.9, 10.2 Hz, 1H), 6.27 (d, J = 15.6 Hz, 1H), 5.74 (d, J = 11.3 Hz, 1H).

### Example 8: Preparation of N-(2-(3,4-dichlorophenyl)-7-(1-methyl-1H-imidazol-4-yl)-1H-indol-5-yl)acrylamide

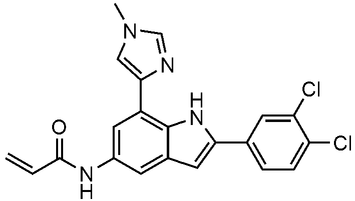

The title compound (3.5 mg, yield: 9%) was obtained in the same manner as in Example 1, except that 1,2-dichloro-4-ethynylbenzene was used instead of 1-chloro-4-ethynylbenzene in step 2 of Example 1.

¹H NMR (500 MHz, DMSO) δ 11.14 (s, 1H), 10.07 (s, 1H), 8.14 (s, 1H), 7.91 (s, 1H), 7.89 (s, 1H), 7.82 (d, J = 8.5 Hz, 1H), 7.77 (s, 1H), 7.74 (d, J = 8.5 Hz, 1H), 7.71 (s, 1H), 7.10 (s, 1H), 6.49 (dd, J = 17.0, 10.0 Hz, 1H), 6.26 (d, J = 17.0 Hz, 1H), 5.73 (d, J = 10.0 Hz, 1H), 3.80 (s, 3H).

### Example 9: Preparation of N-(2-(3,4-difluorophenyl)-7-(1-methyl-1H-imidazol-4-yl)-1H-indol-5-yl)acrylamide

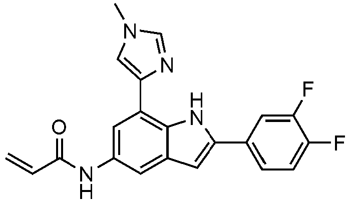

The title compound (2.0 mg, yield: 5%) was obtained in the same manner as in Example 1, except that 1,2-difluoro-4-ethynylbenzene was used instead of 1-chloro-4-ethynylbenzene in step 2 of Example 1.

¹H NMR (500 MHz, CD₃OD) δ 8.03 (d, J = 2.0 Hz, 1H), 7.82 (dd, J = 9.0, 2.0 Hz, 2H), 7.77 (dd, J = 8.5, 2.0 Hz, 1H), 7.67-7.62 (m, 4H), 6.92 (s, 1H), 6.50 (dd, J = 17.0, 10.0 Hz, 1H), 6.40 (dd, J = 17.0, 2.0 Hz, 1H), 5.79 (dd, J = 10.0, 1.5 Hz, 1H), 3.87 (s, 3H).

### Example 10: Preparation of N-(2-(3-chlorophenyl)-7-(1-methyl-1H-imidazol-4-yl)-1H-indol-5-yl)acrylamide

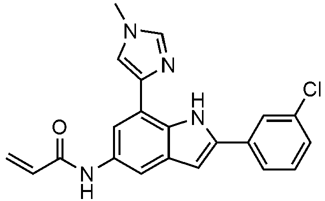

The title compound (11.6 mg, yield: 31%) was obtained in the same manner as in Example 1, except that 1-chloro-3-ethynylbenzene was used instead of 1-chloro-4-ethynylbenzene in step 2 of Example 1.

¹H NMR (500 MHz, DMSO) δ 11.16 (s, 1H), 10.07 (s, 1H), 7.91-7.90 (m, 3H), 7.80-7.77 (m, 2H), 7.70 (s, 1H), 7.55-7.52 (m, 1H), 7.42-7.41 (m, 1H) 7.06 (s, 1H) 6.49 (dd, J = 17.0, 10.4 Hz, 1H), 6.27 (d, J = 16.8 Hz, 1H), 5.74 (d, J = 11.0 Hz, 1H), 3.80 (s, 3H).

### Example 11: Preparation of N-(2-(4-chlorophenyl)-7-(1-(difluoromethyl)-1H-imidazol-4-yl)-1H-indol-5-yl)acrylamide

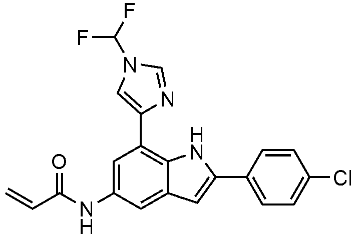

The title compound (9.0 mg, yield: 22%) was obtained in the same manner as in Example 1, except that 1-(difluoromethyl)-4-(tributylstannyl)-1H-imidazole was used instead of 1-methyl-4-(tributylstannyl)-1H-imidazole in step 1 of Example 1.

¹H NMR (500 MHz, DMSO) δ 10.90 (s, 1H), 10.11 (s, 1H), 8.44 (s, 1H), 8.30 (s, 1H), 7.98 (s, 1H), 7.94 (CHF₂, t, J = 60.0 Hz, 1H) 7.91-7.89 (m, 2H), 7.83 (d, J = 1.5 Hz, 1H), 7.57-7.56 (m, 2H), 7.02 (d, J = 1.9 Hz, 1H), 6.50 (dd, J = 16.9, 10.2 Hz, 1H), 6.27 (dd, J = 17.0, 1.7 Hz, 1H), 5.83-5.67 (m, 1H).

### Example 12: Preparation of N-(2-(4-chloro-2-fluorophenyl)-7-(1-methyl-1H-imidazol-4-yl)-1H-indol-5-yl)acrylamide

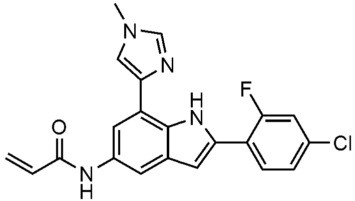

The title compound (4.5 mg, yield: 11%) was obtained in the same manner as in Example 1, except that 4-chloro-1-ethynyl-2-fluorobenzene was used instead of 1-chloro-4-ethynylbenzene in step 2 of Example 1.

¹H NMR (500 MHz, DMSO) δ 11.52 (s, 1H), 10.09 (s, 1H), 8.00 (t, J = 8.5 Hz, 1H), 7.90-7.89 (m, 2H), 7.73-7.70 (m, 2H), 7.67 (dd, J = 11.5, 1.5 Hz, 1H), 7.44 (dd, J = 8.5, 1.5 Hz, 1H), 7.06 (d, J = 2.0 Hz, 1H), 6.50 (dd, J = 17.0, 10.0 Hz, 1H), 6.27 (dd, J = 17.0, 2.0 Hz, 1H), 5.74 (d, J = 10.0, 2.0 Hz, 1H), 3.79 (s, 3H).

### Example 13: Preparation of N-(2-cyclohexenyl-7-(1-methyl-1H-imidazol-4-yl)-1H-indol-5-yl)acrylamide

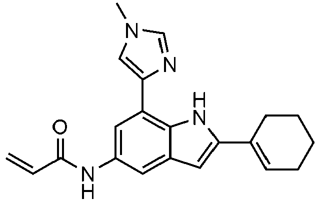

The title compound (11.0 mg, yield: 32%) was obtained in the same manner as in Example 1, except that 1-ethynyl cyclohex-1-ene was used instead of 1-chloro-4-ethynylbenzene in step 2 of Example 1.

¹H NMR (500 MHz, DMSO) δ 10.84 (s, 1H), 10.00 (s, 1H), 7.86 (s, 1H), 7.74 (s, 1H), 7.68 (s, 1H), 7.62 (s, 1H), 6.53- 6.41 (m, 2H), 6.25 (d, J = 17.2 Hz, 2H), 5.72 (d, J = 10.1 Hz, 1H), 3.78 (s, 3H), 2.44-2.46 (m, 2H), 2.25-2.26 (m, 2H), 1.75-1.77 (m, 2H), 1.66-1.68 (m, 2H).

### Example 14: Preparation of N-(2-(4-fluoro-3-methylphenyl)-7-(1-methyl-1H-imidazol-4-yl)-1H-indol-5-yl)acrylamide

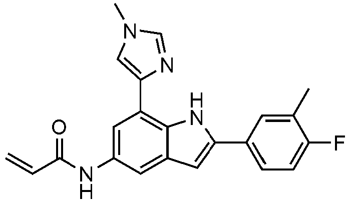

The title compound (6.0 mg, yield: 16%) was obtained in the same manner as in Example 1, except that 4-ethynyl-1-fluoro-2-methylbenzene was used instead of 1-chloro-4-ethynylbenzene in step 2 of Example 1.

¹H NMR (500 MHz, DMSO) δ 11.08 (s, 1H), 10.05 (s, 1H), 7.89 (s, 1H), 7.85 (s, 1H), 7.77-7.73 (m, 2H), 7.67-7.64 (m, 2H), 7.28 (t, J = 9.1 Hz, 1H), 6.88 (d, J = 2.2 Hz, 1H), 6.49 (dd, J = 16.9, 10.1 Hz, 1H), 6.26 (dd, J = 17.0, 1.8 Hz, 1H), 5.79-5.69 (m, 1H), 3.80 (s, 3H), 2.35 (s, 3H).

### Example 15: Preparation of N-(7-(1-methyl-1H-imidazol-4-yl)-2-p-tolyl-1H-indol-5-yl)acrylamide

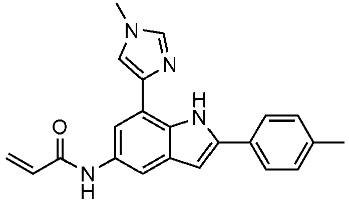

The title compound (3.3 mg, yield: 9%) was obtained in the same manner as in Example 1, except that 1-ethynyl-4-methylbenzene was used instead of 1-chloro-4-ethynylbenzene in step 2 of Example 1.

¹H NMR (500 MHz, DMSO) δ 11.13 (s, 1H), 10.05 (s, 1H), 7.90 (s, 1H), 7.84 (s, 1H), 7.74-7.66 (m, 4H), 7.32 (d, J = 7.5 Hz, 2H), 6.88 (d, J = 2.0 Hz, 1H), 6.49 (d, J = 17.0, 10.0 Hz, 1H), 6.26 (dd, J = 17.0, 1.5 Hz, 1H), 5.73 (dd, J = 10.0, 1.5 Hz, 1H), 3.80 (s, 3H), 2.37 (s, 3H).

### Example 16: Preparation of N-(7-(1-methyl-1H-imidazol-4-yl)-2-(3-(trifluoromethyl)phenyl)-1H-indol-5-yl)acrylamide

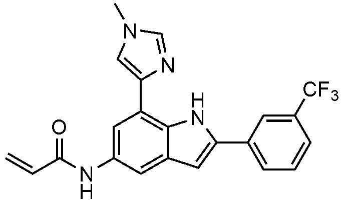

The title compound (3.2 mg, yield: 8%) was obtained in the same manner as in Example 1, except that 1-ethynyl-3-(trifluoromethyl)benzene was used instead of 1-chloro-4-ethynylbenzene in step 2 of Example 1.

¹H NMR (500 MHz, DMSO) δ 11.23 (s, 1H), 10.09 (s, 1H), 8.17 (s, 1H), 8.12 (d, J = 7.5 Hz, 1H), 7.91-7.90 (m, 2H), 7.77 (s, 1H), 7.76-7.70 (m, 3H), 7.14 (d, J = 2.0 Hz, 1H), 6.49 (dd, J = 17.0, 10.5 Hz, 1H), 6.27 (dd, J = 17.0, 1.5 Hz, 1H), 5.74 (dd, J =10.0, 1.5 Hz, 1H), 3.80 (s, 3H).

### Example 17: Preparation of N-(7-(6,7-dihydro-5H-pyrrolo[1,2-a]imidazol-2-yl)-2-(4-fluorophenyl)-1H-indol-5-yl)acrylamide

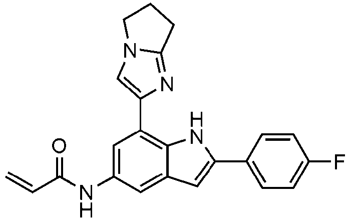

The title compound (6.9 mg, yield: 18%) was obtained in the same manner as in Example 1, except that 1,2-(tributylstannyl)-6,7-dihydro-5H-pyrrole[1,2-a]imidazole was used instead of 1-methyl-4-(tributylstannyl)-1H-imidazole in step 1 of Example 1, and 1-ethynyl-4-fluorobenzene was used instead of 1-chloro-4-ethynylbenzene in step 2 of Example 1.

¹H NMR (500 MHz, DMSO) δ 11.14 (s, 1H), 10.03 (s, 1H), 7.92-7.79 (m, 3H), 7.70 (s, 1H), 7.62 (s, 1H), 7.43-7.29 (m, 2H), 6.90 (s, 1H), 6.49 (dd, J = 17.0, 10.2 Hz, 1H), 6.26 (d, J = 16.6 Hz, 1H), 5.73 (d, J = 11.4 Hz, 1H), 4.14-4.02 (m, 2H), 2.98-2.87 (m, 2H), two protons were missing due to overlapping.

### Example 18: Preparation of N-(2-(4-chlorophenyl)-7-(6,7-dihydro-5H-pyrrolo[1,2-a]imidazol-2-yl)-1H-indol-5-yl)acrylamide

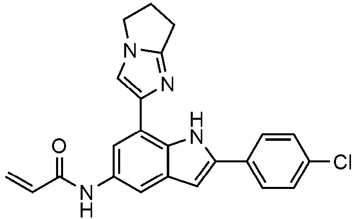

The title compound (6.8 mg, yield: 17%) was obtained in the same manner as in Example 1, except that 2-(tribunylstannyl)-6,7-dihydro-5H-pyrrole[1,2-a]imidazole was used instead of 1-methyl-4-(tributylstannyl)-1H-imidazole in step 1 of Example 1.

¹H NMR (500 MHz, DMSO) δ 11.17 (s, 1H), 10.04 (s, 1H), 7.89 (s, 1H), 7.84 (d, J = 8.5 Hz, 2H), 7.71 (s, 1H), 7.63 (s, 1H), 7.56 (d, J = 8.4 Hz, 2H), 6.98 (s, 1H), 6.49 (dd, J = 16.9, 10.2 Hz, 1H), 6.26 (d, J = 15.2 Hz, 1H), 5.73 (d, J = 11.9 Hz, 1H), 4.14-4.05 (m, 2H), 2.97-2.88 (m, 2H), two protons were missing due to overlapping.

### Example 19: Preparation of N-(7-(1-methyl-1H-imidazol-4-yl)-2-(3-(trifluoromethyl)phenyl)-1H-indol-5-yl)methanesulfonamide

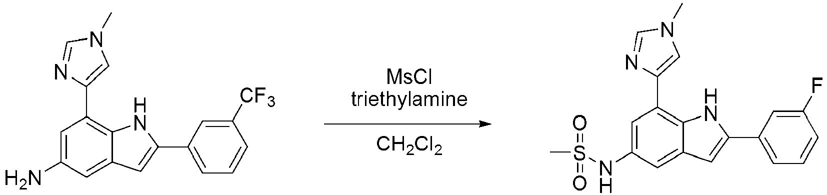

7-(1-Methyl-1H-imidazol-4-yl)-2-(3-(trifluoromethyl)phenyl)-1H-indol-5-amine (0.1 mmol, 0.035 g, 1.0 eq) was dissolved in dichloromethane (1 mL), and then cooled to 0°C. Triethylamine (0.15 mmol, 0.015 g, 1.5 eq) and methanesulfonyl chloride (0.11 mmol, 0.012 g, 1.1 eq) were sequentially added thereto. The reaction mixture was reacted at room temperature for 2 hours. After the reaction was completed, dichloromethane was removed, and purified by a column chromatography to give the title compound (15.0 mg, yield: 35%).

¹H NMR (500 MHz, DMSO) δ 11.32 (s, 1H), 9.36 (s, 1H), 8.16 (s, 1H), 8.12 (d, J = 7.5 Hz, 1H), 7.90 (s, 1H), 7.81 (s, 1H), 7.75-7.21 (m, 2H), 7.40 (d, J = 1.5 Hz, 1H), 7.34 (d, J = 1.5 Hz, 1H), 7.13 (d, J = 2.0 Hz, 1H), 3.80 (s, 3H), 2.93 (s, 3H).

### Example 20: Preparation of N-(2-(4-chloro-3-fluorophenyl)-7-(1-methyl-1H-imidazol-4-yl)-1H-indol-5-yl)acrylamide

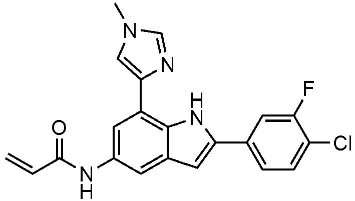

The title compound (16.2 mg, yield: 39%) was obtained in the same manner as in Example 1, except that 1-chloro-4-ethynyl-2-fluorobenzene was used instead of 1-chloro-4-ethynylbenzene in step 2 of Example 1.

¹H NMR (500 MHz, DMSO) δ 11.13 (s, 1H), 10.20 (s, 1H), 8.03-7.99 (m, 2H), 7.96-7.94 (m, 1H), 7.83-7.77 (m, 2H), 7.73-7.70 (m, 2H), 7.15 (s, 1H), 6.50 (dd, J = 17.0, 10.5 Hz, 1H), 6.27 (dd, J = 17.0, 1.5 Hz, 1H), 5.76 (dd, J = 10.0, 1.5 Hz, 1H), 3.93 (s, 3H).

### Example 21: Preparation of N-(2-(4-chlorophenyl)-7-(1-methyl-1H-imidazol-4-yl)-1H-indol-5-yl)methanesulfonamide

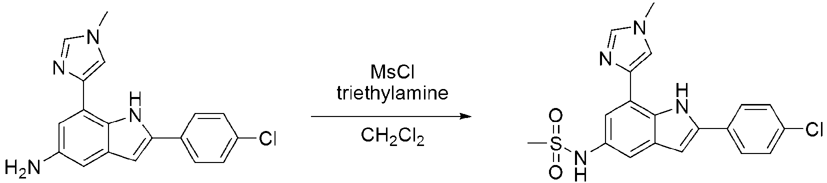

2-(4-Chlorophenyl)-7-(1-methyl-1H-imidazol-4-yl)-1H-indol-5-amine (0.1 mmol, 0.032 g, 1.0 eq) was dissolved in dichloromethane (1 mL), and then cooled to 0°C. Triethylamine (0.15 mmol, 0.015 g, 1.5 eq) and methanesulfonyl chloride (0.11 mmol, 0.012 g, 1.1 eq) were sequentially added thereto. The reaction mixture was reacted at room temperature for 2 hours. After the reaction was completed, dichloromethane was removed, and purified by a column chromatography to give the title compound (19.8 mg, yield: 49%).

¹H NMR (500 MHz, DMSO) δ 11.25(s, 1H), 9.34 (s, 1H), 7.89 (s, 1H), 7.84 (d, J = 8.5 Hz, 2H), 7.79 (s, 1H), 7.57 (d, J = 8.0 Hz, 2H), 7.37 (s, 1H), 7.32 (s, 1H), 6.99 (d, J = 1.5 Hz 1H), 3.80 (s, 3H), 2.92 (s, 3H).

### Example 22: Preparation of N-(2-(4-chlorophenyl)-7-(1-methyl-1H-imidazol-4-yl)-1H-indol-5-yl)-2-fluoroacrylamide

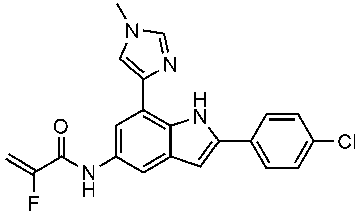

The title compound (6.1 mg, yield: 15%) was obtained in the same manner as in Example 1, except that 2-fluoroacrylic acid was used instead of acrylic acid in step 5 of Example 1.

¹H NMR (500 MHz, DMSO) δ 11.23 (s, 1H), 10.19 (s, 1H), 7.90 (s, 1H), 7.85 (d, J = 8.5 Hz, 2H), 7.82 (s, 1H), 7.75 (m, 2H), 7.57 (d, J = 8.5 Hz, 1H), 7.00 (d, J = 2.0 Hz, 1H), 5.72 (dd, J = 48.0, 3.5 Hz, 1H), 5.41 (dd, J = 5.5, 3.5 Hz, 1H), 3.80 (s, 3H).

### Example 23: Preparation of N-(3-chloro-2-(4-chlorophenyl)-7-(1-methyl-1H-imidazol-4-yl)-1H-indol-5-yl)acrylamide

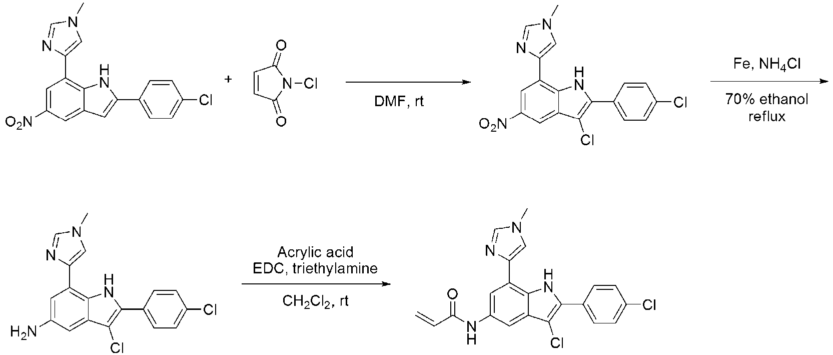

### (Step 1)

2-(4-Chlorophenyl)-7-(1-methyl-1H-imidazol-4-yl)-5-nitro-1H-indole (0.28 mmol, 0.1 g, 1.0 eq) was dissolved in dimethylformamide (1 mL). N-chlorosuccinimide (0.31 mmol, 0.041 g, 1.1 eq) was added thereto, and then reacted overnight at room temperature. After the reaction was completed, distilled water was added, and the solid was filtered to give 3-chloro-2-(4-chlorophenyl)-7-(1-methyl-1H-imidazol-4-yl)-5-nitro-1H-indole (0.060 g, yield: 55%).

### (Step 2)

3-Chloro-2-(4-chlorophenyl)-7-(1-methyl-1H-imidazol-4-yl)-5-nitro-1H-indole (0.15 mmol, 0.060 g, 1.0 eq), iron (1.5 mmol, 0.083 g, 1.0 eq), ammonium chloride (0.15 mmol, 0.008 g, 1.0 eq), and 70% ethanol (10 mL; ethanol: distilled water = 7:3 (v:v)) was sequentially added, and the reactants were reacted at 80°C for 4 hours. After the reaction was completed, iron and ethanol were removed, extracted with an aqueous ammonium chloride solution, and concentrated, and 3-chloro-2-(4-chlorophenyl)-7-(1-methyl-1H-imidazol-4-yl)-1H-indol-5-amine was used in the next reaction without separation and purification.

### (Step 3)

3-Chloro-2-(4-chlorophenyl)-7-(1-methyl-1H-imidazol-4-yl)-1H-indol-5-amine (0.1 mmol, 0.035 g, 1.0 eq), N-(3-dimethylaminopropyl)-N-ethylcarbodiimide hydrochloride (0.2 mmol, 0.038 g, 2.0 eq), and dichloromethane (1 mL) were sequentially added to a flask. Triethylamine (0.2 mL) and acrylic acid (0.1 mmol, 0.0072, 1.0 eq) were added thereto, and then reacted overnight at room temperature. After the reaction was completed, dichloromethane was removed, and purified by a column chromatography to give the title compound (3.7 mg, yield: 9%).

¹H NMR (500 MHz, DMSO) δ 11.20 (s, 1H), 10.22 (s, 1H), 7.95 (s, 1H), 7.95-7.90 (m, 2H), 7.87 (s, 1H), 7.82-7.80 (m, 2H), 7.67-7.65 (m, 2H), 6.48 (d, J = 10.4 Hz, 1H), 6.28 (d, J = 17.3 Hz, 1H), 5.76 (d, J = 10.0 Hz, 1H), 3.79 (s, 3H).

### Example 24: Preparation of (E)-N-(2-(4-chlorophenyl)-7-(1-methyl-1H-imidazol-4-yl)-1H-indol-5-yl)-2-cyano-3-phenylacrylamide

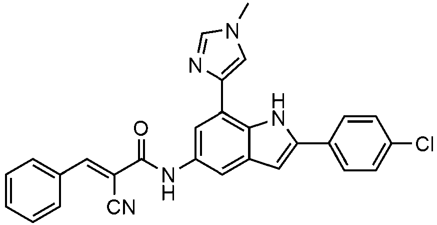

The title compound (19.8 mg, yield: 41%) was obtained in the same manner as in Example 1, except that (E)-2-cyano-3-phenylacrylic acid was used instead of acrylic acid in step 5 of Example 1.

¹H NMR (500 MHz, DMSO) δ 11.25 (s, 1H), 8.67 (s, 1H), 7.99-7.97(m, 2H), 7.73 (d, J = 8.0 Hz, 2H), 7.60 (s, 1H), 7.52-7.51 (m, 3H), 7.46-7.42 (m, 5H), 6.86 (d, J = 2.0 Hz, 1H), 3.81 (s, 3H).

### Example 25: Preparation of N-(2-(4-chlorophenyl)-7-(1-methyl-1H-imidazol-4-yl)-1H-indol-5-yl)but-2-ynamide

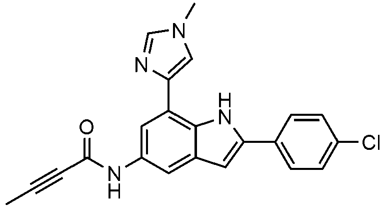

The title compound (15.4 mg, yield: 39%) was obtained in the same manner as in Example 1, except that but-2-ynoic acid was used instead of acrylic acid in step 5 of Example 1.

¹H NMR (500 MHz, DMSO) δ 11.19 (s, 1H), 10.48 (s, 1H), 7.89 (s, 1H), 7.84 (d, J = 7.0 Hz, 2H), 7.73 (s, 1H), 7.71 (s, 1H), 7.66 (s, 1H), 7.57 (d, J = 7.0 Hz, 2H), 6.97 (s, 1H), 5.77 (s, 3H), 3.80 (s, 3H).

### Example 26: Preparation of N-(2-(3,4-dichlorophenyl)-7-(1-methyl-1H-imidazol-4-yl)-1H-indol-5-yl)ethanesulfonamide

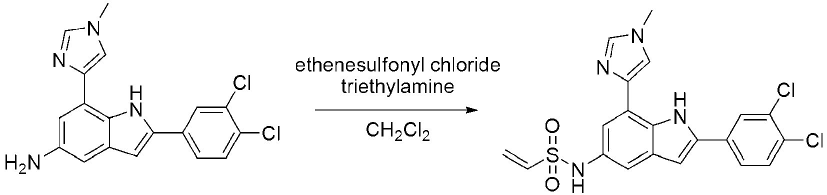

2-(3,4-Dichlorophenyl)-7-(1-methyl-1H-imidazol-4-yl)-1H-indol-5-amine (0.1 mmol, 0.036 g, 1.0 eq) was dissolved in dichloromethane (1 mL), and then cooled to 0°C. Triethylamine (0.15 mmol, 0.015 g, 1.5 eq) and ethenesulfonyl chloride (0.11 mmol, 0.014 g, 1.1 eq) were sequentially added thereto. The reaction mixture was reacted at room temperature for 2 hours. After the reaction was completed, dichloromethane was removed, and purified by a column chromatography to give the title compound (21.1 mg, yield: 51%).

¹H NMR (500 MHz, DMSO) δ 11.20 (s, 1H), 9.64 (s, 1H), 8.12 (d, J = 2.0 Hz, 1H), 7.82-7.74 (m, 3H), 7.34 (d, J = 1.5 Hz, 1H), 7.25 (s, 1H), 7.08 (d, J = 2.5 Hz, 1H), 6.75 (dd, J = 16.0, 9.5 Hz, 1H), 6.02-5.95 (m, 2H), 3.80 (s, 3H).

### Example 27: Preparation of 1-(2-(3,4-dichlorophenyl)-7-(1-methyl-1H-imidazol-4-yl)-1H-indol-5-yl)-1H-pyrrole-2,5-dione

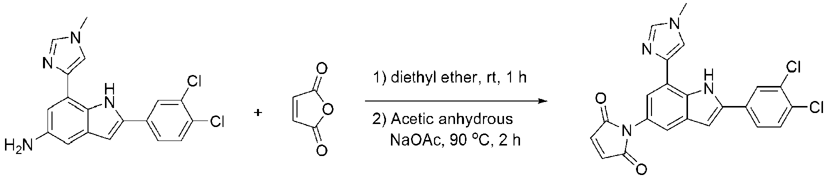

2-(3,4-Dichlorophenyl)-7-(1-methyl-1H-imidazol-4-yl)-1H-indol-5-amine (0.1 mmol, 0.036 g, 1.0 eq), diethyl ether (0.3 mL) and maleic anhydride (0.1 mmol, 0.0098 g, 1.0 eq) were sequentially added to a sealed tube, and then reacted at room temperature for 1 hour. After the reaction was completed, diethyl ether was removed. Sodium acetate (0.05 mmol, 0.0041 g, 0.5 eq) and acetic anhydride (0.3 mL) were added to the concentrated residue, and then reacted at 90°C for 2 hours. After the reaction was completed, the reaction mixture was extracted with an aqueous potassium carbonate solution and ethyl acetate, concentrated, and purified by a column chromatography to give the title compound (8.9 mg, yield: 20%).

¹H NMR (500 MHz, DMSO) δ 8.17 (d, J = 2.0 Hz, 1H), 7.91 (s, 1H), 7.86-7.84 (m, 2H), 7.78 (d, J = 8.5 Hz, 1H), 7.41-7.38 (m, 2H), 7.21 (s, 1H), 7.18 (d, J = 2.0 Hz, 1H), 3.79 (s, 3H).

### Example 28: Preparation of N-(2-(4-chlorophenyl)-7-(1-methyl-1H-pyrazol-3-yl)-1H-indol-5-yl)acrylamide

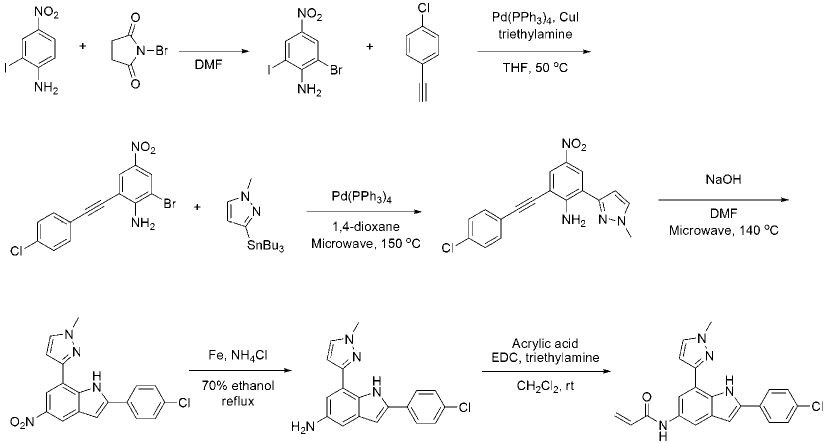

### (Step 1)

2-lodo-4-nitroaniline (37.8 mmol, 10 g, 1.0 eq) was dissolved in dimethylformamide (74 mL), and then cooled to 0°C. N-bromosuccinimide (41.6 mmol, 7.4 g 1.1 eq) was added thereto, and then reacted at room temperature for 5 hours. After the reaction was completed, distilled water (50 mL) was added thereto, and stirred for 10 minutes. The yellow solid was filtered to give 2-bromo-6-iodo-4-nitroaniline (10 g, yield: 77%).

### (Step 2)

2-Bromo-6-iodo-4-nitroaniline (1.44 mmol, 0.5 g, 1.0 eq), Pd(PPh₃)₄ (0.072 mmol, 0.083 g, 0.05 eq), copper iodide (0.14 mmol, 0.026 g, 0.1 eq), triethylamine (4.32 mmol, 0.6 mL, 3.0 eq) and tetrahydrofuran (10 mL) were sequentially added, and then ultrasonic degassing was carried out under a nitrogen atmosphere for 10 minutes. 1-Chloro-4-ethynylbenzene (1.59 mmol, 0.21 g, 1.1 eq) was added thereto, and reacted overnight at 40°C. After the reaction was completed, the reaction mixture was extracted with ethyl acetate and ammonium chloride aqueous solution, concentrated, and then solidified with tert-butyl methyl ether to give 2-bromo-6-((4-chlorophenyl)ethynyl)-4-nitroaniline (0.3 g, yield: 59%).

### (Step 3)

In a sealed tube, 2-bromo-6-((4-chlorophenyl)ethynyl)-4-nitroaniline (0.77 mmol, 0.3 g, 1.0 eq) was dissolved in 1,4-dioxane (10 mL). 1-Methyl-3-(tributylstannyl)-1H-pyrazole (1.0 mmol, 0.37 g, 1.3 eq), and Pd(PPh₃)₄ (0.077 mmol, 0.088 g, 0.1 eq) were sequentially added thereto, and then reacted in a microwave reactor at 150°C for 1 hour. After the reaction was completed, the reaction mixture was extracted with ethyl acetate and ammonium chloride aqueous solution, concentrated, and then solidified with tert-butyl methyl ether to give 2-((4-chlorophenyl)ethynyl)-6-(1-methyl-1H-pyrazol-3-yl)-4-nitroaniline (0.14 g, yield: 51%).

### (Step 4)

2-((4-Chlorophenyl)ethynyl)-6-(1-methyl-1H-pyrazol-3-yl)-4-nitroaniline (0.39 mmol, 0.14 g, 1.0 eq), sodium hydroxide (1.81 mmol, 0.072 g, 4.6 eq) and dimethylformamide (10 mL) were sequentially added to a microwave vial, and then reacted at 150°C for 20 minutes. After the reaction was completed, the reaction mixture was extracted with ethyl acetate and ammonium chloride aqueous solution, concentrated, and then solidified with tert-butyl methyl ether to give 2-(4-chlorophenyl)-7-(1-methyl-1H-pyrazol-3-yl)-5-nitro-1H-indole (0.1 g, yield: 72%).

### (Step 5)

2-(4-Chlorophenyl)-7-(1-methyl-1H-pyrazole-3-yl)-5-nitro-1H-indole (0.28 mmol, 0.1 g, 1.0 eq), iron (2.8 mmol, 0.15 g, 1.0 eq), ammonium chloride (0.28 mmol, 0.014 g, 1.0 eq), and 70% ethanol (5 mL; ethanol: distilled water = 7:3 (v:v)) were sequentially added. The reactants were reacted at 70°C for 3 hours. After the reaction was completed, iron and ethanol were removed, extracted with an aqueous ammonium chloride solution, and concentrated, and 2-(4-chlorophenyl)-7-(1-methyl-1H-pyrazol-3-yl)-1H-indol-5-amine was used in the next reaction without separation and purification.

### (Step 6)

2-(4-Chlorophenyl)-7-(1-methyl-1H-pyrazol-3-yl)-1H-indol-5-amine (0.1 mmol, 0.032 g, 1.0 eq), N-(3-dimethylaminopropyl)-N-ethylcarbodiimide hydrochloride (0.2 mmol, 0.038 g, 2.0 eq), and dichloromethane (1 mL) were sequentially added to a flask. Triethylamine (0.1 mL) and acrylic acid (0.11 mmol, 0.0079 g, 1.1 eq) were added thereto, and then reacted overnight at room temperature. After the reaction was completed, dichloromethane was removed, and purified by a column chromatography to give the title compound (9.0 mg, yield: 25%).

¹H NMR (500 MHz, DMSO) δ 10.77 (s, 1H), 10.10 (s, 1H), 8.00 (s, 1H), 7.92-7.88 (m, 3H), 7.71 (s, 1H), 7.57-7.55 (m, 2H), 7.03 (d, J = 2.1 Hz, 1H), 6.76 (d, J = 2.1 Hz, 1H), 6.49 (dd, J = 16.9, 10.1 Hz, 1H), 6.27 (d, J = 17.0 Hz, 1H), 5.75 (d, J = 10.1 Hz, 1H), 4.07 (s, 3H).

### Example 29: Preparation of N-(2-(3,4-dichlorophenyl)-7-(1-methyl-1H-pyrazol-3-yl)-1H-indol-5-yl)acrylamide

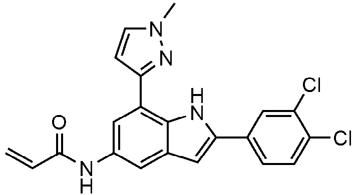

The title compound (14.8 mg, yield: 36%) was obtained in the same manner as in Example 28, except that 1,2-dichloro-4-ethynylbenzene was used instead of 1-chloro-4-ethynylbenzene in step 2 of Example 28.

¹H NMR (500 MHz, DMSO) δ 10.82 (s, 1H), 10.11 (s, 1H), 8.20 (s, 1H), 8.02 (s, 1H), 7.89-7.88 (m, 2H), 7.78-7.67 (m, 2H), 7.14 (s, 1H), 6.76 (s, 1H), 6.48 (dd, J = 17.0, 10.2 Hz, 1H), 6.31-6.24 (m, 1H), 5.79-5.72 (m, 1H), 4.06 (s, 3H).

### Example 30: Preparation of N-(2-(3,4-dichlorophenyl)-7-(pyridin-2-yl)-1H-indol-5-yl)acrylamide

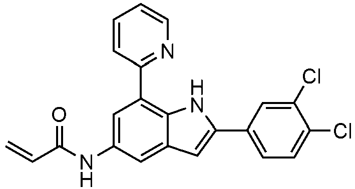

The title compound (11.4 mg, yield: 28%) was obtained in the same manner as in Example 28, except that 1,2-dichloro-4-ethynylbenzene was used instead of 1-chloro-4-ethynylbenzene in step 2 of Example 28, and 2-(tributylstannyl)pyridine was used instead of 1-methyl-3-(tributylstannyl)-1H-pyrazole in step 3 of Example 28.

¹H NMR (500 MHz, DMSO) δ 11.57 (s, 1H), 10.19 (s, 1H), 8.88 (d, J = 4.5 Hz, 1H), 8.25 (br s, 1H), 8.12 (s, 1H), 8.05-8.00 (m, 3H), 7.94 (d, J = 8.2 Hz, 1H), 7.73 (d, J = 8.4 Hz, 1H), 7.47-7.43 (m, 1H), 7.18 (s, 1H), 6.50 (dd, J = 16.9, 10.1 Hz, 1H), 6.29 (d, J = 16.9 Hz, 1H), 5.77 (d, J = 10.1 Hz, 1H).

### Example 31: Preparation of N-(2-(4-fluorophenyl)-7-(pyridin-2-yl)-1H-indol-5-yl)acrylamide

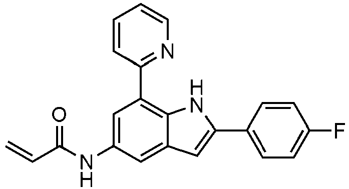

The title compound (9.6 mg, yield: 27%) was obtained in the same manner as in Example 28, except that 1-ethynyl-4-fluorobenzene was used instead of 1-chloro-4-ethynylbenzene in step 2 of Example 28, and 2-(tributylstannyl)pyridine was used instead of 1-methyl-3-(tributylstannyl)-1H-pyrazole in step 3 of Example 28.

¹H NMR (500 MHz, DMSO) δ 11.56 (s, 1H), 10.18 (s, 1H), 8.88 (d, J = 4.6 Hz, 1H), 8.09 (s, 1H), 8.06 - 7.94 (m, 5H), 7.47 - 7.42 (m, 1H), 7.36 - 7.33 (m, 2H), 7.01 (d, J = 2.3 Hz, 1H), 6.50 (dd, J = 16.9, 10.1 Hz, 1H), 6.29 (d, J = 16.9 Hz, 1H), 5.77 (d, J = 11.9 Hz, 1H).

### Example 32: Preparation of N-(2-(4-fluorophenyl)-7-(1-methyl-1H-pyrazol-3-yl)-1H-indol-5-yl)acrylamide

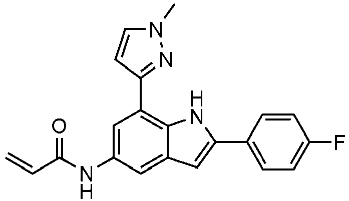

The title compound (7.9 mg, yield: 22%) was obtained in the same manner as in Example 28, except that 1-ethynyl-4-fluorobenzene was used instead of 1-chloro-4-ethynylbenzene in step 2 of Example 28.

¹H NMR (500 MHz, DMSO) δ 10.73 (s, 1H), 10.10 (s, 1H), 7.98 (s, 1H), 7.94-7.91 (m, 2H), 7.89 (d, J = 2.2 Hz, 1H), 7.70 (d, J = 1.6 Hz, 1H), 7.37-7.33 (m, 2H), 6.97 (d, J = 2.2 Hz, 1H), 6.76 (d, J = 2.2 Hz, 1H), 6.49 (dd, J = 16.9, 10.1 Hz, 1H), 6.27 (dd, J = 17.0, 1.8 Hz, 1H), 5.79-5.71 (m, 1H), 4.06 (s, 3H).

### Example 33: Preparation of N-(2-(2,4-difluorophenyl)-7-(1-methyl-1H-pyrazol-3-yl)-1H-indol-5-yl)acrylamide

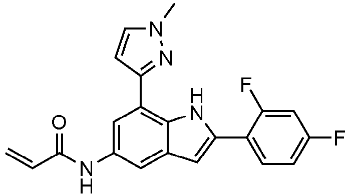

The title compound (8.6 mg, yield: 23%) was obtained in the same manner as in Example 28, except that 1-ethynyl-2,4-difluorobenzene was used instead of 1-chloro-4-ethynylbenzene in step 2 of Example 28.

¹H NMR (500 MHz, DMSO) δ 11.04 (s, 1H), 10.13 (s, 1H), 8.09-8.04 (m, 1H), 8.02 (s, 1H), 7.90 (d, J = 2.0 Hz, 1H), 7.74 (d, J = 1.5 Hz, 1H), 7.52-7.47 (m, 1H), 7.27 (td, J = 8.5, 2.5 Hz, 1H), 7.05 (d, J = 2.0 Hz, 1H), 6.76 (d, J = 2.5 Hz, 1H), 6.49 (dd, J = 17.0, 10.0 Hz, 1H), 6.28 (dd, J = 17.0, 2.0 Hz, 1H), 5.75 (dd, J = 10.0, 2.0 Hz, 1H), 4.03 (s, 3H).

### Example 34: Preparation of N-(2-(5-chloropyridin-2-yl)-7-(1-methyl-1H-imidazol-4-yl)-1H-indol-5-yl)acrylamide

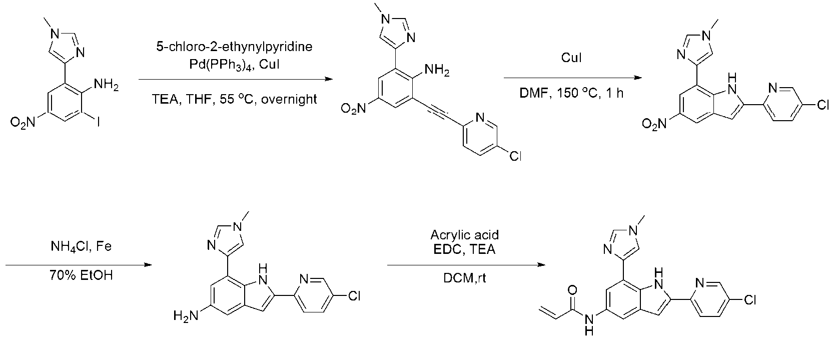

### (Step 1)

In a sealed tube, 2-iodo-6-(1-methyl-1H-imidazol-4-yl)-4-nitroaniline (1.5 mmol, 0.52 g, 1.0 eq) was dissolved in THF (2.5 mL) and TEA (2.5 mL). Pd(PPh₃)₄ (0.15 mmol, 173.3 mg, 0.1 eq) and Cul (0.15 mmol, 28.6 mg, 0.1 eq) were added, and then 5-chloro-2-ethynylpyridine (3.0 mmol, 0.41 g, 2.0 eq) was added, and reacted overnight at 55°C. After the reaction was completed, the temperature was cooled to room temperature. Tert-butyl methyl ether (5 mL) was added, and the solid was filtered to give 2-((5-chloropyridin-2-yl)ethynyl)-6-(1-methyl-1H-imidazol-4-yl)-4-nitroaniline (0.49 g, yield: 92%).

### (Step 2)

In a sealed tube, 2-((5-chloropyridin-2-yl)ethynyl)-6-(1-methyl-1H-imidazol-4-yl)-4-nitroaniline (1.4 mmol, 0.48 g, 1.0 eq) was dissolved in DMF (7.0 mL), and then CuI (0.14 mmol, 26.7 mg, 0.1 eq) was added thereto, and reacted at 150°C for 1 hour. After the reaction was completed, the temperature was cooled to room temperature. The reaction mixture was extracted with a saturated aqueous sodium chloride solution and ethyl acetate, concentrated, and then separated and purified by a column chromatography to give 2-(5-chloropyridin-2-yl)-7-(1-methyl-1H-imidazol-4-yl)-5-nitro-1H-indole (0.22 g, yield: 44%). Steps 3 and 4 were carried out in the same manner as in Example 1 to give the title compound (12.8 mg, yield: 31%).

¹H NMR (500 MHz, DMSO) δ 9.93 (s, 1H), 8.66 (s, 1H), 8.02 (dd, J = 8.0, 2.5 Hz, 1H), 7.83 (d, J = 8.5 Hz, 1H), 7.79 (s, 1H), 7.76 (d, J = 2.5 Hz, 1H), 7.59 (d, J = 2.0 Hz, 1H), 7.40 (s 1H), 6.67 (s, 2H), 6.40 (dd, J = 16.0, 10.0 Hz, 1H), 6.23 (dd, J = 16.0, 1.5 Hz, 1H), 5.72 (dd, J = 10.0, 1.5 Hz, 1H), 3.75 (s, 3H).

### Example 35: Preparation of 2-(4-fluorophenyl)-7-(1-methyl-1H-imidazol-4-yl)-1H-indole-5-carboxylic acid

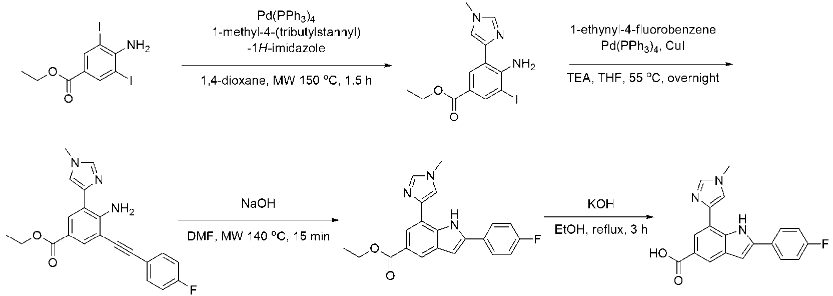

### (Step 1)

Ethyl-4-amino-3,5-diiodobenzoate (2.0 mmol, 0.83 g, 1.0 eq) was dissolved in 1,4-dioxane (10 mL), and then 1-methyl-4-(tributylstanyl)-1H-imidazole (2.1 mmol, 0.78 g, 1.05 eq), Pd(PPh₃)₄ (0.4 mmol, 0.46 g, 0.2 eq) were sequentially added to a sealed tube, and then reacted in a microwave reactor at 140°C for 1.5 hours. After the reaction was completed, the solvent was removed, and the obtained concentrated residue was separated and purified by a column chromatography to give ethyl 4-amino-3-iodo-5-(1-methyl-1H-imidazol-4-yl)benzoate (0.32 g, yield: 44%).

### (Step 2)

In a sealed tube, ethyl 4-amino-3-iodo-5-(1-methyl-1H-imidazol-4-yl)benzoate (0.5 mmol, 186 mg, 1.0 eq) was dissolved in tetrahydrofuran (0.8 mL), and then 1-ethynyl-4-fluorobenzene (0.8 mmol, 96 mg, 1.6 eq), Pd(PPh₃)₄ (0.05 mmol, 58 mg, 0.1 eq), Cul (0.03 mmol, 4.8 mg, 0.05 eq) and triethylamine (0.8 mL) were added thereto, and reacted overnight at 55°C. After the reaction was completed, the temperature was cooled to room temperature. Water (10 mL) and ethyl acetate (5 mL) were added, and the target compound was extracted with the organic layer, dried, and concentrated to give ethyl 4-amino-3-((4-fluorophenyl)ethynyl)-5-(1-methyl-1H-imidazol-4-yl)benzoate (0.15 g, yield: 81%).

### (Step 3)

In a sealed tube, ethyl 4-amino-3-((4-fluorophenyl)ethynyl)-5-(1-methyl-1H-imidazol-4-yl)benzoate (0.4 mmol, 0.15 g, 1.0 eq) was added to and dissolved in dimethylacetamide (2 mL), and then sodium hydroxide (2.0 mmol, 80 mg, 5.0 eq) was added, and reacted in a microwave reactor at 140°C for 15 minutes. Saturated aqueous sodium chloride solution (50 mL) and ethyl acetate (15 mL) were added and extracted, and the obtained organic layer was dried, and then the solvent was removed. The result was separated and purified by a column chromatography to give ethyl 2-(4-fluorophenyl)-7-(1-methyl-1H-imidazol-4-yl)-1H-indole-5-carboxylate (70 mg, yield: 48%).

### (Step 4)

Ethyl 2-(4-fluorophenyl)-7-(1-methyl-1H-imidazol-4-yl)-1H-indole-5-carboxylate (0.1 mmol, 0.036 g, 1.0 eq), 70% ethanol (0.5 mL; ethanol: distilled water = 7:3 (v:v)), and potassium hydroxide (0.2 mmol, 11.2 mg, 2.0 eq) were sequentially added to a sealed tube, and the mixture was then refluxed for 2 hours. After the reaction was completed, the temperature was cooled to room temperature. Acetic acid (0.2 mL) and purified water (1 mL) were added to the reaction solution, and the resulting solid was filtered to give the title compound (21.5 mg, yield: 19%).

¹H NMR (500 MHz, DMSO) δ 11.30 (s, 1H), 8.06 (s, 1H), 8.01 (s, 1H), 7.88 (s, 1H), 7.84 (m, 2H), 7.79 (s, 1H), 7.35 (m, 2H), 6.94 (s, 1H), 3.79 (s, 3H).

### Example 36: Preparation of 7-(1-methyl-1H-imidazol-4-yl)-2-(4-(trifluoromethyl)phenyl)-1H-indole-5-carboxylic acid

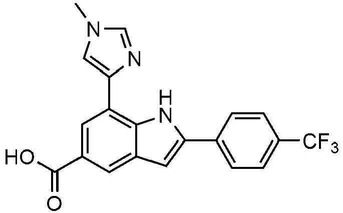

The title compound (21.5 mg, yield: 56%) was obtained in the same manner as in Example 35, except that 1-ethynyl-4-(trifluoromethyl)benzene was used instead of 1-ethynyl-4-fluorobenzene in step 2 of Example 35.

¹H NMR (500 MHz, DMSO) δ 11.71 (s, 1H), 8.16 (s, 1H), 8.12-8.03 (m, 3H), 7.99 (s, 1H), 7.93 (s, 1H), 7.88 (d, J = 8.2 Hz, 2H), 7.29 (d, J = 1.7 Hz, 1H), 3.80 (s, 3H).

### Example 37: Preparation of 2-(4-chlorophenyl)-7-(1-methyl-1H-imidazol-4-yl)-1H-indole-5-carboxylic acid

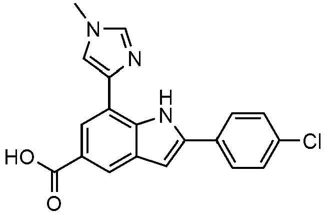

The title compound (8.6 mg, yield: 24%) was obtained in the same manner as in Example 35, except that 1-chloro-4-ethynylbenzene was used instead of 1-ethynyl-4-fluorobenzene in step 2 of Example 35.

¹H NMR (500 MHz, DMSO) δ 12.50 (br, s, 1H), 11.60 (s, 1H), 8.12 (s, 1H), 8.07 (s, 1H), 7.99 (s, 1H), 7.93 (s, 1H), 7.88 (d, J = 8.5 Hz, 2H), 7.60 (d, J = 8.5 Hz, 2H), 7.15 (d, J = 1.5 Hz, 1H), 3.80 (s, 3H).

### Example 38: Preparation of 2-(3,4-dichlorophenyl)-7-(1-methyl-1H-imidazol-4-yl)-1H-indole-5-carboxylic acid

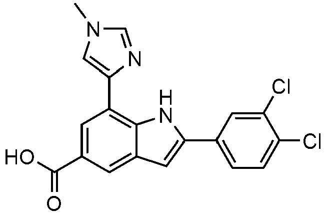

The title compound (6.7 mg, yield: 17%) was obtained in the same manner as in Example 35, except that 1,2-dichloro-4-ethynylbenzene was used instead of 1-ethynyl-4-fluorobenzene in step 2 of Example 35.

¹H NMR (500 MHz, DMSO) δ 12.56 (br, s, 1H), 11.58 (s, 1H), 8.17 (d, J = 1.5 Hz, 1H), 8.13 (s, 1H), 8.09 (s, 1H), 7.98 (s, 1H), 7.92 (s, 1H), 7.84 (dd, J = 8.5, 1.5 Hz, 1H), 7.78 (d, J = 8.5 Hz, 1H), 7.26 (d, J = 2.0 Hz, 1H), 3.80 (s, 3H).

### Example 39: Preparation of 2-(4-chloro-3-fluorophenyl)-7-(1-methyl-1H-imidazol-4-yl)-1H-indole-5-carboxylic acid

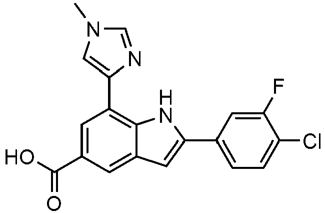

The title compound (22.5 mg, yield: 60%) was obtained in the same manner as in Example 35, except that 1-chloro-4-ethynyl-2-fluorobenzene was used instead of 1-ethynyl-4-fluorobenzene in step 2 of Example 35.

¹H NMR (500 MHz, DMSO) δ 12.59 (br, s, 1H), 11.59 (s, 1H), 8.13 (s, 1H), 8.09 (s, 1H), 7.99-7.97 (m, 2H), 7.92 (s, 1H), 7.76-7.71 (m, 2H), 7.25 (s, 1H), 3.80 (s, 3H).

### Example 40: Preparation of 2-(4-chlorophenyl)-N-methyl-7-(1-methyl-1H-imidazol-4-yl)-1H-indole-5-carboxylamide

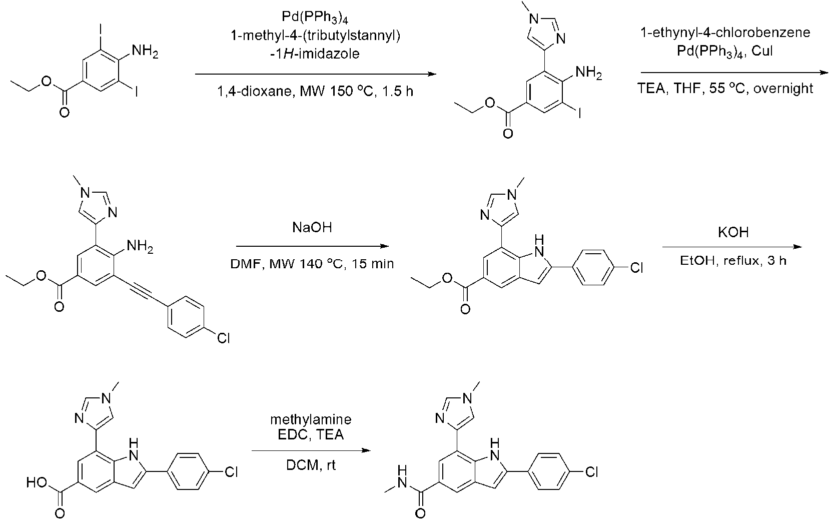

### (Step 1)

In a sealed tube, ethyl-4-amino-3,5-diiodobenzoate (2.0 mmol, 0.83 g, 1.0 eq) was dissolved in 1,4-dioxane (10 mL), and then 1-methyl-4-(tributylstannyl)-1H-imidazole (2.1 mmol, 0.78 g, 1.05 eq), and Pd(PPh₃)₄ (0.4 mmol, 0.46 g, 0.2 eq) were sequentially added thereto, and then reacted in a microwave reactor at 140°C for 1.5 hours. After the reaction was completed, the solvent was removed, the obtained concentrated residue was separated and purified by a column chromatography to give ethyl 4-amino-3-iodo-5-(1-methyl-1H-imidazol-4-yl)benzoate (0.32 g, yield: 44%).

### (Step 2)

In a sealed tube, ethyl 4-amino-3-iodo-5-(1-methyl-1H-imidazol-4-yl)benzoate (0.5 mmol, 186 mg, 1.0 eq) was dissolved in tetrahydrofuran (0.8 mL), and then 1-ethynyl-4-chlorobenzene (0.8 mmol, 96 mg, 1.6 eq), Pd(PPh₃)₄ (0.05 mmol, 58 mg, 0.1 eq), Cul (0.03 mmol, 4.8 mg, 0.05 eq) and triethylamine (0.8 mL) were added thereto, and reacted overnight at 55°C. After the reaction was completed, the temperature was cooled to room temperature. Water (10 mL) and ethyl acetate (5 mL) were added, and the target compound was extracted with the organic layer, dried, and concentrated to give ethyl 4-amino-3-((4-chlorophenyl)ethynyl)-5-(1-methyl-1H-imidazol-4-yl)benzoate (0.16 g, yield: 84%).

### (Step 3)

In a sealed tube, ethyl 4-amino-3-((4-chlorophenyl)ethynyl)-5-(1-methyl-1H-imidazol-4-yl)benzoate (0.4 mmol, 0.15 g, 1.0 eq) was added to and dissolved in dimethylacetamide (2 mL), and then sodium hydroxide (2.0 mmol, 80 mg, 5.0 eq) was added, and reacted in a microwave reactor at 140°C for 15 minutes. Saturated aqueous sodium chloride solution (50 mL) and ethyl acetate (15 mL) were added, extracted and then the obtained organic layer was dried and the solvent was removed. The result was separated and purified by a column chromatography to give ethyl 2-(4-chlorophenyl)-7-(1-methyl-1H-imidazol-4-yl)-1H-indole-5-carboxylate (81 mg, yield: 53%).

### (Step 4)

Ethyl 2-(4-chlorophenyl)-7-(1-methyl-1H-imidazol-4-yl)-1H-indole-5-carboxylate (0.2 mmol, 0.080 g, 1.0 eq), 70% ethanol (0.5 mL; ethanol: distilled water = 7:3 (v:v)), and potassium hydroxide (0.2 mmol, 22.4 mg, 2.0 eq) were sequentially added to a sealed tube, and then the mixture was refluxed for 2 hours. After the reaction was completed, the temperature was cooled to room temperature. Acetic acid (0.2 mL) and purified water (1 mL) were added to the reaction solution, and the resulting solid was filtered to give 2-(4-chlorophenyl)-7-(1-methyl-1H-imidazol-4-yl)-1H-indole-5-carboxylic acid (36 mg, yield: 51%).

### (Step 5)

2-(4-Chlorophenyl)-7-(1-methyl-1H-imidazol-4-yl)-1H-indole-5-carboxylic acid (0.1 mmol, 0.035 g, 1.0 eq), N-(3-dimethylaminopropyl)-N-ethylcarbodiimide hydrochloride (0.2 mmol, 0.038 g, 2.0 eq), and dichloromethane (1 mL) were sequentially added to a flask. Triethylamine (0.1 mL) and acrylic acid (0.11 mmol, 0.0079 g, 1.1 eq) were added, and then reacted overnight at room temperature. After the reaction was completed, dichloromethane was removed, and purified by a column chromatography to give the title compound (10.3 mg, yield: 28%).

¹H NMR (500 MHz, DMSO) δ 11.47 (s, 1H), 8.38-8.35 (m, 1H), 8.00-7.99 (m, 1H), 7.92 (s, 1H), 7.89-7.87 (m, 3H), 7.60-7.58 (m, 2H), 7.11 (s, 1H), 3.81 (s, 3H), 2.83 (s, 3H).

### Example 41: Preparation of N-(7-(1-methyl-1H-imidazol-4-yl)-2-(3,4,5-trichlorophenyl)-1H-indol-5-yl)acrylamide

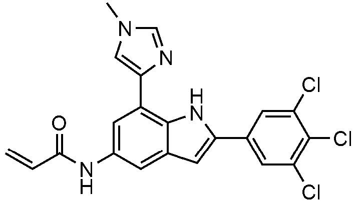

The title compound (16.8 mg, yield: 19%) was obtained in the same manner as in Example 1, except that 1,2,3-trichloro-5-ethynylbenzene was used instead of 1-chloro-4-ethynylbenzene in step 2 of Example 1.

¹H NMR (500 MHz, DMSO) δ 11.10 (s, 1H), 10.10 (s, 1H), 8.16 (s, 2H), 7.94 (s, 1H), 7.89 (s, 1H), 7.79 (s, 1H), 7.72 (s, 1H), 7.20 (d, J = 1.5 Hz, 1H), 6.49 (dd, J = 17.0, 10.0 Hz, 1H), 6.26 (d, J = 17.0 Hz, 1H), 5.74 (d, J = 10.0 Hz, 1H), 3.8 (s, 3H).

### Example 42: Preparation of N-(2-(3-fluorophenyl)-7-(1-methyl-1H-imidazol-4-yl)-1H-indol-5-yl)acrylamide

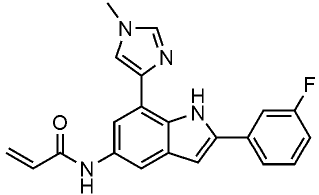

The title compound (13.5 mg, yield: 36%) was obtained in the same manner as in Example 1, except that 1-ethynyl-3-fluorobenzene was used instead of 1-chloro-4-ethynylbenzene in step 2 of Example 1.

¹H NMR (500 MHz, DMSO) δ 11.16 (s, 1H), 10.08 (s, 1H), 7.89 (s, 2H), 7.77 (s, 1H), 7.70-7.65 (m, 3H), 7.55 (dd, J = 8.0 Hz 1H), 7.21-7.17 (m, 1H), 7.04 (d, J =2.5 Hz, 1H), 6.49 (dd, J = 17.0, 10.0 Hz, 1H), 6.27 (dd, J = 17.0, 2.0 Hz, 1H), 5.74 (dd, J = 10.0, 2.0 Hz, 1H), 3.80 (s, 3H).

### Example 43: Preparation of 2-(3,4-difluorophenyl)-7-(1-methyl-1H-imidazol-4-yl)-1H-indole-5-carboxylic acid

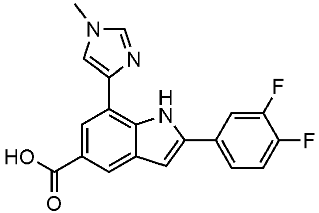

The title compound (23.4 mg, yield: 74%) was obtained in the same manner as in Example 35, except that 4-ethynyl-1,2-difluorobenzene was used instead of 1-ethynyl-4-fluorobenzene in step 2 of Example 35.

¹H NMR (500 MHz, DMSO) δ 11.52 (s, 1H), 8.11 (s, 1H), 8.08 (s, 1H), 8.01-7.96 (m, 2H), 7.91 (s, 1H), 7.70-7.69 (m, 1H), 7.61 (dd, J = 9.0 Hz, 1H), 7.15 (d, J = 2.0 Hz, 1H), 3.80 (s, 3H).

### Example 44: Preparation of N-(2-(3-chloro-4-fluorophenyl)-7-(1-methyl-1H-imidazol-4-yl)-1H-indol-5-yl)acrylamide

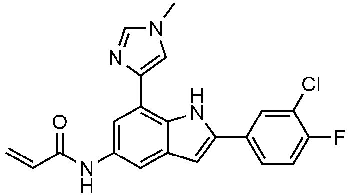

The title compound (10.8 mg, yield: 27%) was obtained in the same manner as in Example 1, except that 2-chloro-4-ethynyl-1-fluorobenzene was used instead of 1-chloro-4-ethynylbenzene in step 2 of Example 1.

¹H NMR (500 MHz, DMSO) δ 11.09 (s, 1H), 10.06 (s, 1H), 8.08 (d, J = 5.0 Hz, 1H), 7.88 (d, J = 7.0 Hz, 2H), 7.83 (s, 1H), 7.76 (s, 1H), 7.69 (s, 1H), 7.56-7.52 (m, 1H),7.01 (s, 1H), 7.01 (s, 1H), 6.49 (dd, J = 17.0, 10.0 Hz, 1H), 6.26 (d, J = 10.0 Hz, 1H), 3.80 (s, 3H).

### Example 45: Preparation of N-(2-(2,4-dichlorophenyl)-7-(1-methyl-1H-imidazol-4-yl)-1H-indol-5-yl)acrylamide

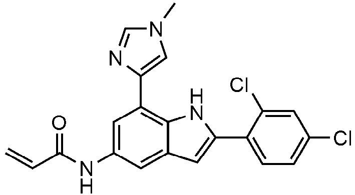

The title compound (12.0 mg, yield: 27%) was obtained in the same manner as in Example 1, except that 2,4-dichloro-1-ethynylbenzene was used instead of 1-chloro-4-ethynylbenzene in step 2 of Example 1.

¹H NMR (500 MHz, DMSO) δ 1.50 (s, 1H), 10.09 (s, 1H), 7.91 (s, 1H), 7.89-7.84 (m, 2H), 7.80 (d, J = 2.0 Hz, 1H), 7.72 (d, J = 1.5 Hz, 1H), 7.59-7.57 (m, 1H), 6.94 (d, J = 2.0 Hz, 1H), 6.50 (dd, J = 17.0, 10.0 Hz, 1H), 6.27 (dd, J = 17.0, 2.0 Hz, 1H), 5.74 (dd, J = 10.0, 2.0 Hz, 1H), 3.78 (s, 3H).

### Example 46: Preparation of N-(2-(4-chloro-3,5-difluorophenyl)-7-(1-methyl-1H-imidazol-4-yl)-1H-indol-5-yl)acrylamide

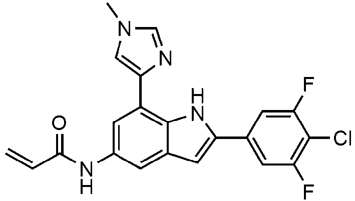

The title compound (36.2 mg, yield: 18%) was obtained in the same manner as in Example 1, except that 2-chloro-5-ethynyl-1,3-difluorobenzene was used instead of 1-chloro-4-ethynylbenzene in step 2 of Example 1.

¹H NMR (500 MHz, DMSO) δ 11.10 (s, 1H), 10.11 (s, 1H), 7.94 (s, 1H), 7.89-7.88 (m, 3H), 7.79 (s, 1H), 7.72 (s, 1H), 7.18 (s, 1H), 6.49 (dd, J = 17.0, 10.0 Hz, 1H), 6.26 (d, J = 17.0 Hz, 1H), 5.74 (dd, J = 10.0 Hz, 1H), 3.81 (s, 3H).

### Example 47: Preparation of N-(2-(4-chloro-2,6-difluorophenyl)-7-(1-methyl-1H-imidazol-4-yl)-1H-indol-5-yl)acrylamide

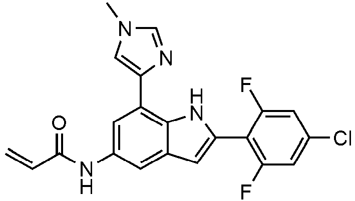

The title compound (34.7 mg, yield: 10%) was obtained in the same manner as in Example 1, except that 5-chloro-2-ethynyl-1,3-difluorobenzene was used instead of 1-chloro-4-ethynylbenzene in step 2 of Example 1.

¹H NMR (500 MHz, DMSO) δ 11.65 (s, 1H), 10.11 (s, 1H), 7.90 (s, 2H), 7.74 (dd, J = 7.0, 1.5 Hz, 1H), 7.60 (d, J = 9.5 Hz, 1H), 7.00 (s, 1H), 6.50 (d, J = 17.0, 10.0 Hz, 1H), 6.27 (dd, J = 17.0, 2.0 Hz, 1H), 5.74 (dd, J = 10.0, 2.0 Hz, 1H), 3.79 (s, 3H).

### Example 48: Preparation of N-(7-(1-methyl-1H-imidazol-4-yl)-2-(4-(trifluoromethoxy)phenyl)-1H-indol-5-yl)acrylamide

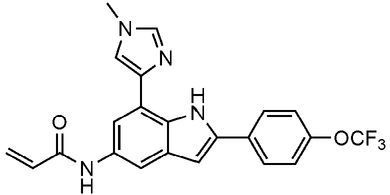

The title compound (38.0 mg, yield: 89%) was obtained in the same manner as in Example 1, except that 1-ethynyl-4-(trifluoromethoxy)benzene was used instead of 1-chloro-4-ethynylbenzene in step 2 of Example 1.

¹H NMR (500 MHz, DMSO) δ 11.17 (s, 1H), 10.06 (s, 1H), 7.94 (d, J = 8.5 Hz, 1H), 7.88 (s, 2H), 7.76 (s, 1H), 7.70 9s, 1H), 7.49 (d, J = 8.5 Hz, 1H), 6.98 (s, 1H), 6.49 (dd, J = 17.0, 10.0 Hz, 1H), 6.26 (d, J = 17.0 Hz, 1H), 5.73 (d, J = 10.0 Hz, 1H), 3.80 (s, 3H).

### Example 49: Preparation of N-(2-(4-chlorophenyl)-7-(4-methylthiazol-2-yl)-1H-indol-5-yl)acrylamide

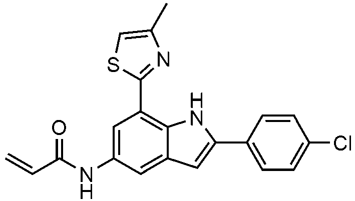

The title compound (15.1 mg, yield: 38%) was obtained in the same manner as in Example 28, except that 4-methyl-2-(tributylstannyl)thiazole was used instead of 1-methyl-3-(tributylstannyl)-1H-pyrazole in step 3 of Example 28.

¹H NMR (500 MHz, DMSO) δ 11.08 (s, 1H), 10.23 (s, 1H), 8.12 (d, J = 1.7 Hz, 1H), 7.97 (d, J = 1.8 Hz, 1H), 7.91 (d, J = 8.5 Hz, 2H), 7.58 (d, J = 8.5 Hz, 2H), 7.40 (s, 1H), 7.10 (d, J = 2.3 Hz, 1H), 6.47 (dd, J = 17.0, 10.1 Hz, 1H), 6.36 - 6.23 (m, 1H), 5.81 - 5.71 (m, 1H), 2.60 (s, 3H).

### Example 50: Preparation of N-(7-(1-ethyl-1H-imidazol-4-yl)-2-(3,4,5-trifluorophenyl)-1H-indol-5-yl)acrylamide

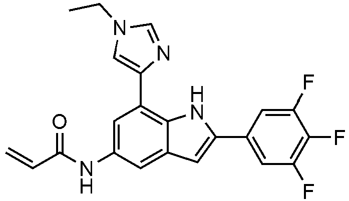

The title compound (15.0 mg, yield: 36%) was obtained in the same manner as in Example 28, except that 5-ethynyl-1,2,3-trifluorobenzene was used instead of 1-chloro-4-ethynylbenzene in step 2 of Example 28, and 1-ethyl-4-(tributylstannyl)-1H-imidazole was used instead of 1-methyl-3-(tributylstannyl)-1H-pyrazole in step 3 of Example 28.

¹H NMR (500 MHz, CDCl3) δ 7.87 (d, J = 1.9 Hz, 1H), 7.65 (s, 1H), 7.57 (s, 1H), 7.46 (s, 1H), 7.42 - 7.33 (m, 4H), 6.74 (d, J = 2.6 Hz, 1H), 6.49 (d, J = 16.8 Hz, 1H), 6.32 (dd, J = 16.8, 10.2 Hz, 1H), 5.80 (d, J = 10.1 Hz, 1H), 4.10 (q, J = 7.3 Hz, 2H), 1.56 (t, J = 7.3 Hz, 3H).

### Example 51: Preparation of N-(7-(1-ethyl-1H-imidazol-4-yl)-2-(4-fluorophenyl)-1H-indol-5-yl)acrylamide

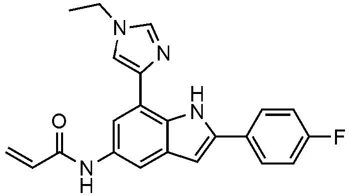

The title compound (25.2 mg, yield: 66%) was obtained in the same manner as in Example 28, except that 1-ethynyl-4-fluorobenzene was used instead of 1-chloro-4-ethynylbenzene in step 2 of Example 28, and 1-ethyl-4-(tributylstannyl)-1H-imidazole was used instead of 1-methyl-3-(tributylstannyl)-1H-pyrazole in step 3 of Example 28.

¹H NMR (500 MHz, DMSO) δ 11.14 (s, 1H), 10.05 (s, 1H), 7.96 (s, 1H), 7.94 - 7.81 (m, 4H), 7.71 (d, J = 1.8 Hz, 1H), 7.35 (t, J = 8.7 Hz, 2H), 6.91 (d, J = 2.3 Hz, 1H), 6.49 (dd, J = 17.0, 10.1 Hz, 1H), 6.26 (dd, J = 17.0, 2.1 Hz, 1H), 5.74 (dd, J = 10.1, 2.1 Hz, 1H), 4.13 (q, J = 7.3 Hz, 2H), 1.46 (t, J = 7.3 Hz, 3H).

### Example 52: Preparation of N-(2-(4-bromophenyl)-7-(1-methyl-1H-imidazol-4-yl)-1H-indole-5-yl)acrylamide

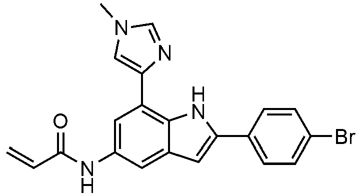

The title compound (38.0 mg, yield: 89%) was obtained in the same manner as in Example 1, except that 1-bromo-4-ethynylbenzene was used instead of 1-chloro-4-ethynylbenzene in step 2 of Example 1.

¹H NMR (500 MHz, DMSO) δ 11.16 (s, 1H), 10.07 (s, 1H), 7.92 - 7.86 (m, 2H), 7.83 - 7.75 (m, 3H), 7.73 - 7.67 (m, 3H), 6.99 (d, J = 2.4 Hz, 1H), 6.49 (dd, J = 16.9, 10.1 Hz, 1H), 6.35 - 6.12 (m, 1H), 5.74 (dd, J = 10.1, 2.1 Hz, 1H), 3.80 (s, 3H).

### Experimental Example 1: Activity inhibition test of transcription factor TEAD

The transcription factor TEAD activity of the compounds prepared in Examples was measured by the ONE-Glo^{™} Luciferase assay (Promega, Catalog #E6110) method using the Hippo Pathway TEAD reporter-MCF7 recombinant cell line (BPS Bioscience, Catalog #60618).

Specifically, the TEAD Reporter-MCF7 cell line contains the firefly luciferase gene whose expression is regulated under the control of TEAD response elements in the MCF7, which is a human breast cancer cell line. In the cell line, a non-phosphorylated YAP/TAZ exists in the nucleus in a non-stressed condition, which continuously induces expression of the luciferase reporter.

TEAD Reporter-MCF7 cells were prepared in 100 µL of cell culture medium (MEM medium, 10% FBS, 1% P/S, 400 µg/mL Geneticin, 1% N-ethyl acetate A, 1 mM NA pyruvate, 10 µg/mL Insulin) in a white clear bottom 96-well microplate. The prepared plate was incubated in a 5% CO₂ incubator at 37°C for 24 hours, and then the previously prepared compounds of Examples were finally treated at concentrations of 0.0005, 0.005, 0.05, 0.5 and 5 µM, which were repeated three times for all treatment solutions. The plates treated with the compounds of Examples were incubated in a 5% CO₂ incubator at 37°C for 24 hours. 100 µL/well of the substrate aqueous solution containing luciferin was added to a white clear-bottom 96 well microplate, thereby initiating an enzyme reaction. The reaction was performed at room temperature for 5 minutes, and the luminescence (integration time 1000 ms) was measured using a Flexstation3 multi-mode microplate reader. In accordance with the instructions for the ONE-Glo^{™} Luciferase Assay, the luciferase enzyme activity, which represents the activity of the transcription factor TEAD, was measured by a chemiluminescence method, and the inhibitory activity of the compound according to the present disclosure was calculated. The results of each compound were analyzed using Microsoft Excel, and the IC₅₀ values were calculated by GraphPad Prism software. The results are shown in Table 1 below.

### Experimental Example 2: Cell growth inhibition test

The cell growth for the compounds prepared in Examples was measured by CellTiter-Glo^{®} Luminescednt Cell Viability (Promega, Catalog #G7571) method using NCI-H226 (addexbio), NCI-H28 (Korea Cell Line Bank) cell lines. This evaluation method is a method in which when luciferase enzyme is activated by ATP flowing out from living cells, it reacts with a luciferin substrate, and the enzyme activity at this time is measured to confirm cell viability.

Specifically, NCI-H226, and NCI-H28 cells were prepared in 100 µL of cell culture medium (RPMI medium, 10% FBS, 1% P/S, 4.5 g/L D-Glucose, 2.383 g/L HEPES Buffer, L-Glutamine, 1.5 g/L Sodium Bicarbonate, 110 mg/L Sodium pyruvate) in a white clear bottom 96-well microplate. The prepared plate was incubated in a 5% CO₂ incubator at 37°C for 24 hours, and then the previously prepared compounds of Examples were finally treated at concentrations of 0.0005, 0.005, 0.05, 0.5 and 5 µM, which was repeated three times for all treatment solutions. The plates treated with the compounds of Examples were incubated in a 5% CO₂ incubator at 37°C. 100 µL/well of the substrate aqueous solution containing luciferin was added to a white clear-bottom 96 well microplate, thereby initiating an enzyme reaction. The reaction was performed at room temperature in the dark for 10 minutes, and the luminescence was measured using a Flexstation3 multi-mode microplate reader. In accordance with the instructions for the CellTiter-Glo^{™} Luminescednt Cell Viability, the luciferase enzyme activity, which represents the amount of ATP, was measured by a chemiluminescence method, and the inhibitory activity of the compounds according to the present disclosure was calculated. The results of each compound were analyzed using Microsoft Excel, and the IC₅₀ values were calculated using GraphPad Prism software. The results are shown in Table 1 below.

**[Table 1]**

| Example No. | TEAD IC₅₀ (nM) | Growth IC₅₀ (µM) H226 | Growth IC₅₀ (µM) H28 | Example No. | TEAD IC₅₀ (nM) | Growth IC₅₀ (µM) H226 | Growth IC₅₀ (µM) H28 |
|---|---|---|---|---|---|---|---|
| 1 | 0.0094 | 0.0210 | >5 | 30 | 0.1260 | 0.3386 | 0.553 |
| 2 | 0.0390 | 0.0650 | 4.140 | 31 | 0.0300 | 0.012 | 1.767 |
| 3 | 0.0035 | 0.0025 | 1.530 | 32 | 0.0230 | 0.0076 | 1.357 |
| 4 | 0.0300 | 0.0100 | 4.400 | 33 | 0.0510 | 0.0243 | 4.914 |
| 5 | 0.0064 | 0.0042 | >5 | 35 | 0.1000 | 0.1367 | >5 |
| 6 | 0.0059 | 0.0044 | 4.000 | 37 | 0.0260 | 0.1345 | 0.759 |
| 8 | 0.1100 | 0.0570 | 0.950 | 39 | 0.0360 | 0.0219 | >5 |
| 9 | 0.0200 | 0.0053 | 2.950 | 42 | 0.0970 | 0.0045 | 2.080 |
| 10 | 0.0600 | 0.0200 | 2.469 | 43 | 0.0540 | 0.0078 | >5 |
| 12 | 0.0790 | 0.0225 | 2.800 | 44 | 0.0370 | 0.0217 | 1.900 |
| 13 | 0.0460 | 0.0223 | 3.500 | 46 | 0.0900 | 0.0790 | 1.360 |
| 14 | 0.0490 | 0.0251 | 2.300 | 50 | 0.0270 | 0.0154 | >5 |
| 19 | 0.1900 | 0.2099 | 0.438 | 51 | 0.0480 | 0.0076 | 2.540 |
| 21 | 0.2360 | >5 | 2.221 | 52 | 0.3460 | 0.0580 | 0.868 |
| 28 | 0.0150 | 0.0084 | 2.300 | | | | |

### Experimental Example 3: CTGF, CYR61 Analysis Test

NCI-H226 (AddexBio) human mesothelioma cells were cultured in a 6-well cell culture plate at a density of 5x10⁵ cells per well. After a stabilization process for 24 hours, Example 1 was treated at a concentration of 500 nM, and cells treated with the same amount of DMSO were set as a control. 24 hours after treatment, cells were collected to secure RNA for target gene expression analysis.

RNA was extracted from cells using a TRIzol solution (Ambion). Once cell lysis and digestion were completed, chloroform was added to each sample and the homogenate was separated into aqueous and organic phases by centrifugation.

The expression of ctgf (connective tissue growth factor) and cyr61 (Cysteine-rich angiogenic inducer 61), which are genes regulated by YAP-TEAD, and gapdh (Glyceraldehyde 3-phosphate dehydrogenase), which is a housekeeping gene, were quantified by qRT-PCR analysis using the Maxima SYBR Green/Fluorescein qPCR Master Mix and the primers for each gene. The ctgf, cyr61 and gapdh cycle threshold (Ct) values of cell cDNA samples were determined, and ctgf, cyr61 expression was normalized to gapdh.

The expression of each gene in the experimental group treated with Example 1 was normalized relative to the control treated with DMSO. FIG. 1 shows the expression regulatory properties of two TEAD target genes in Example 1 using real-time PCR.

## Claims

1. A compound represented by the following Chemical Formula 1, or a pharmaceutically acceptable salt thereof: 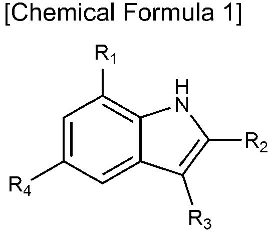 in Chemical Formula 1,
R₁ is a C₆₋₁₀ aryl; or a 5- or 6-membered heterocycle containing 1 to 4 heteroatoms each independently selected from the group consisting of N, O and S,
wherein the R₁ is unsubstituted; or substituted with halogen, a C₁₋₄ alkyl, a C₁₋₄ thioalkyl, a C₁₋₄ haloalkyl, a C₁₋₄ alkoxy, a C₁₋₄ thioalkoxy, a C₁₋₄ haloalkoxy, a C₂₋₄ alkenyl, a C₂₋₄ alkynyl, a C₃₋₆ cycloalkyl, amino, nitro, cyano, a (C₁₋₄ alkyl)amino, or a di(C₁₋₄ alkyl)amino; or is fused with a C₃₋₆ cycloalkyl ring,
R₂ is a C₆₋₁₀ aryl; a C₃₋₆ cycloalkenyl; or a 5- or 6-membered heterocycle containing 1 to 4 heteroatoms each independently selected from the group consisting of N, O and S,
wherein the R₂ is unsubstituted; or substituted with 1 to 3 substituents each independently selected from the group consisting of halogen, a C₁₋₄ alkyl, a C₁₋₄ thioalkyl, a C₁₋₄ haloalkyl, a C₁₋₄ alkoxy, a C₁₋₄ thioalkoxy, a C₁₋₄ haloalkoxy, a C₂₋₄ alkenyl, a C₂₋₄ alkynyl, a C₃₋₆ cycloalkyl, amino, nitro, cyano, a (C₁₋₄ alkyl)amino, and a di(C₁₋₄ alkyl)amino,
R₃ is hydrogen or halogen,
R₄ is -NHCO-R', -NHSO₂-R', -COO-R', -CONH-R', or 1H-pyrrole-2,5-dione-1-yl,
wherein the R' is hydrogen, a C₁₋₄ alkyl, a C₂₋₄ alkynyl, or a substituent represented by the following Chemical Formula 2: 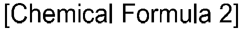 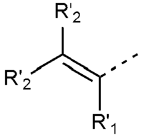
in Chemical Formula 2,
R'₁ is hydrogen, halogen, or cyano, and
R'₂ is each independently hydrogen, a C₆₋₁₀ aryl, -CH₂-NH₂, -CH₂-NH(C₁₋₄ alkyl), or -CH₂-N(C₁₋₄ alkyl)₂.

2. The compound or the pharmaceutically acceptable salt thereof according to claim 1, wherein:
R₁ is phenyl, pyridinyl, pyrimidinyl, imidazolyl, pyrazolyl, triazolyl, tetrazolyl, oxadiazolyl, pyrrolidinyl, imidazolyl, pyrazolyl, oxazolyl, isoxazolyl, thiazolyl, or isothiazolyl,
wherein the R₁ is unsubstituted; or substituted with halogen, a C₁₋₄ alkyl, a C₁₋₄ thioalkyl, a C₁₋₄ haloalkyl, a C₁₋₄ alkoxy, a C₁₋₄ haloalkoxy, amino, nitro, cyano, a (C₁₋₄ alkyl)amino, or a di(C₁₋₄ alkyl)amino, or is fused with a C₃₋₆ cycloalkyl ring.

3. The compound or the pharmaceutically acceptable salt thereof according to claim 1, wherein:
R₁ is imidazolyl, imidazolyl fused with cyclopentane, pyrazolyl, or pyridinyl,
wherein the R₁is unsubstituted; or substituted with methyl, ethyl, fluoromethyl, difluoromethyl, or trifluoromethyl.

4. The compound or the pharmaceutically acceptable salt thereof according to claim 1, wherein:
R₂ is phenyl, pyridinyl, pyrimidinyl, imidazolyl, pyrazolyl, triazolyl, tetrazolyl, oxadiazolyl, pyrrolidinyl, imidazolyl, pyrazolyl, oxazolyl, isoxazolyl, thiazolyl, or isothiazolyl,
wherein the R₂ is unsubstituted; or substituted with halogen, a C₁₋₄ alkyl, a C₁₋₄ thioalkyl, a C₁₋₄ haloalkyl, a C₁₋₄ alkoxy, a C₁₋₄ haloalkoxy, amino, nitro, cyano, a (C₁₋₄ alkyl)amino, or a di(C₁₋₄ alkyl)amino.

5. The compound or the pharmaceutically acceptable salt thereof according to claim 1, wherein:
R₂ is phenyl, pyridinyl, or cyclohexenyl,
wherein the R₂ is unsubstituted; or substituted with 1 to 3 substituents each independently selected from the group consisting of fluoro, chloro, brormo, methyl, trifluoromethyl, and trifluoromethoxy.

6. The compound or the pharmaceutically acceptable salt thereof according to claim 1, wherein:
R₃ is hydrogen, or chloro.

7. The compound or the pharmaceutically acceptable salt thereof according to claim 1, wherein:
R₄ is -NHCO-R', -NHSO₂-R', -COO-R', -CONH-R', or 1H-pyrrole-2,5-dione-1-yl;
wherein the R' is hydrogen; a C₁₋₄ alkyl; a C₂₋₄ alkenyl which is unsubstituted, or substituted with 1 or 2 substituents each independently selected from the group consisting of phenyl, fluoro, and cyano; or a C₂₋₄ alkynyl.

8. The compound or the pharmaceutically acceptable salt thereof according to claim 1, wherein:
R₄ is -NHCO-CH=CH₂, -NHCO-CF=CH₂, -NHCO-C(CN)=CH(phenyl), -NHCO-C≡C-CH₃, -NHSO₂-CH₃, -NHSO₂-CH=CH₂, -COOH, -CONH-CH₃, or 1H-pyrrole-2,5-dione-1-yl,

9. The compound or the pharmaceutically acceptable salt thereof according to claim 1, wherein:
the Chemical Formula 1 is represented by the following Chemical Formula 3: 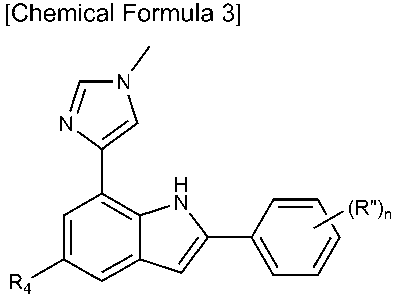
in Chemical Formula 3,
R" is each independently hydrogen, halogen, a C₁₋₄ alkyl, or a C₁₋₄ haloalkyl,
n is an integer of 1 to 3, and
R₄ is -NHCO-CH=CH₂, or -COOH.

10. The compound or the pharmaceutically acceptable salt thereof according to claim 1, wherein the compound represented by Chemical Formula 1 is any one selected from the group consisting of:
1) N-(2-(4-chlorophenyl)-7-(1-methyl-1H-imidazol-4-yl)-1H-indol-5-yl)acrylamide,
2) N-(7-(1-methyl-1H-imidazol-4-yl)-2-(4-(trifluoromethyl)phenyl)-1H-indol-5-yl)acrylamide,
3) N-(2-(4-fluorophenyl)-7-(1-methyl-1H-imidazol-4-yl)-1H-indol-5-yl)acrylamide,
4) N-(7-(1-methyl-1H-imidazol-4-yl)-2-phenyl-1 H-indol-5-yl)acrylamide,
5) N-(2-(2,4-difluorophenyl)-7-(1-methyl-1H-imidazol-4-yl)-1H-indol-5-yl)acrylamide,
6) N-(7-(1-methyl-1H-imidazol-4-yl)-2-(3,4,5-trifluorophenyl)-1H-indol-5-yl)acrylamide,
7) N-(7-(1-(difluoromethyl)-1H-imidazol-4-yl)-2-(4-fluorophenyl)-1H-indol-5-yl)acrylamide,
8) N-(2-(3,4-dichlorophenyl)-7-(1-methyl-1H-imidazol-4-yl)-1H-indol-5-yl)acrylamide,
9) N-(2-(3,4-difluorophenyl)-7-(1-methyl-1H-imidazol-4-yl)-1H-indol-5-yl)acrylamide,
10) N-(2-(3-chlorophenyl)-7-(1-methyl-1H-imidazol-4-yl)-1H-indol-5-yl)acrylamide,
11) N-(2-(4-chlorophenyl)-7-(1-(difluoromethyl)-1H-imidazol-4-yl)-1H-indol-5-yl)acrylamide,
12) N-(2-(4-chloro-2-fluorophenyl)-7-(1-methyl-1H-imidazol-4-yl)-1H-indol-5-yl)acrylamide,
13) N-(2-cyclohexenyl-7-(1-methyl-1H-imidazol-4-yl)-1H-indol-5-yl)acrylamide,
14) N-(2-(4-fluoro-3-methylphenyl)-7-(1-methyl-1H-imidazol-4-yl)-1H-indol-5-yl)acrylamide,
15) N-(7-(1-methyl-1H-imidazol-4-yl)-2-p-tolyl-1H-indol-5-yl)acrylamide,
16) N-(7-(1-methyl-1H-imidazol-4-yl)-2-(3-(trifluoromethyl)phenyl)-1H-indol-5-yl)acrylamide,
19) N-(7-(1-methyl-1H-imidazol-4-yl)-2-(3-(trifluoromethyl)phenyl)-1H-indol-5-yl)methanesulfonamide,
20) N-(2-(4-chloro-3-fluorophenyl)-7-(1-methyl-1H-imidazol-4-yl)-1H-indol-5-yl)acrylamide,
21) N-(2-(4-chlorophenyl)-7-(1-methyl-1H-imidazol-4-yl)-1H-indol-5-yl)methanesulfonamide,
22) N-(2-(4-chlorophenyl)-7-(1-methyl-1H-imidazol-4-yl)-1H-indol-5-yl)-2-fluoroacrylamide,
23) N-(3-chloro-2-(4-chlorophenyl)-7-(1-methyl-1H-imidazol-4-yl)-1H-indol-5-yl)acrylamide,
24) (E)-N-(2-(4-chlorophenyl)-7-(1-methyl-1H-imidazol-4-yl)-1H-indol-5-yl)-2-cyano-3-phenylacrylamide,
25) N-(2-(4-chlorophenyl)-7-(1-methyl-1H-imidazol-4-yl)-1H-indol-5-yl)but-2-ynamide,
26) N-(2-(3,4-dichlorophenyl)-7-(1-methyl-1H-imidazol-4-yl)-1H-indol-5-yl)ethenesulfonamide,
27) 1-(2-(3,4-dichlorophenyl)-7-(1-methyl-1H-imidazol-4-yl)-1H-indol-5-yl)-1H-pyrrole-2,5-dione,
28) N-(2-(4-chlorophenyl)-7-(1-methyl-1H-pyrazol-3-yl)-1H-indol-5-yl)acrylamide,
29) N-(2-(3,4-dichlorophenyl)-7-(1-methyl-1H-pyrazol-3-yl)-1H-indol-5-yl)acrylamide,
30) N-(2-(3,4-dichlorophenyl)-7-(pyridin-2-yl)-1H-indol-5-yl)acrylamide,
31) N-(2-(4-fluorophenyl)-7-(pyridin-2-yl)-1H-indol-5-yl)acrylamide,
32) N-(2-(4-fluorophenyl)-7-(1-methyl-1H-pyrazol-3-yl)-1H-indol-5-yl)acrylamide,
33) N-(2-(2,4-difluorophenyl)-7-(1-methyl-1H-pyrazol-3-yl)-1H-indol-5-yl)acrylamide,
34) N-(2-(5-chloropyridin-2-yl)-7-(1-methyl-1H-imidazol-4-yl)-1H-indol-5-yl)acrylamide,
35) 2-(4-fluorophenyl)-7-(1-methyl-1H-imidazol-4-yl)-1H-indole-5-carboxylic acid,
36) 7-(1-methyl-1H-imidazol-4-yl)-2-(4-(trifluoromethyl)phenyl)-1H-indole-5-carboxylic acid,
37) 2-(4-chlorophenyl)-7-(1-methyl-1H-imidazol-4-yl)-1H-indole-5-carboxylic acid,
38) 2-(3,4-dichlorophenyl)-7-(1-methyl-1H-imidazol-4-yl)-1H-indole-5-carboxylic acid,
39) 2-(4-chloro-3-fluorophenyl)-7-(1-methyl-1H-imidazol-4-yl)-1H-indole-5-carboxylic acid,
40) 2-(4-chlorophenyl)-N-methyl-7-(1-methyl-1H-imidazol-4-yl)-1H-indole-5-carboxamide,
41) N-(7-(1-methyl-1H-imidazol-4-yl)-2-(3,4,5-trichlorophenyl)-1H-indol-5-yl)acrylamide,
42) N-(2-(3-fluorophenyl)-7-(1-methyl-1H-imidazol-4-yl)-1H-indol-5-yl)acrylamide,
43) 2-(3,4-difluorophenyl)-7-(1-methyl-1H-imidazol-4-yl)-1H-indole-5-carboxylic acid,
44) N-(2-(3-chloro-4-fluorophenyl)-7-(1-methyl-1H-imidazol-4-yl)-1H-indol-5-yl)acrylamide,
45) N-(2-(2,4-dichlorophenyl)-7-(1-methyl-1H-imidazol-4-yl)-1H-indol-5-yl)acrylamide,
46) N-(2-(4-chloro-3,5-difluorophenyl)-7-(1-methyl-1H-imidazol-4-yl)-1H-indol-5-yl)acrylamide,
47) N-(2-(4-chloro-2,6-difluorophenyl)-7-(1-methyl-1H-imidazol-4-yl)-1H-indol-5-yl)acrylamide,
48) N-(7-(1-methyl-1H-imidazol-4-yl)-2-(4-(trifluoromethoxy)phenyl)-1H-indol-5-yl)acrylamide,
49) N-(2-(4-chlorophenyl)-7-(4-methylthiazol-2-yl)-1H-indol-5-yl)acrylamide,
50) N-(7-(1-ethyl-1H-imidazol-4-yl)-2-(3,4,5-trifluorophenyl)-1H-indol-5-yl)acrylamide,
51) N-(7-(1-ethyl-1H-imidazol-4-yl)-2-(4-fluorophenyl)-1H-indol-5-yl)acrylamide, and
52) N-(2-(4-bromophenyl)-7-(1-methyl-1H-imidazol-4-yl)-1H-indol-5-yl)acrylamide.

11. A pharmaceutical composition for the prevention or treatment of cancer or tumors, comprising the compound according to any one of claims 1 to 10, or a pharmaceutically acceptable salt thereof as an active ingredient.
